(19) Europäisches Patentamt / European Patent Office / Office européen des brevets

(11) **EP 3 526 582 B1**

(12) **FASCICULE DE BREVET EUROPEEN**

(45) Date de publication et mention
de la délivrance du brevet:
**01.06.2022 Bulletin 2022/22**

(21) Numéro de dépôt: **16790672.6**

(22) Date de dépôt: **14.10.2016**

(51) Classification Internationale des Brevets (IPC):
**G01N 15/00** *(2006.01)* **B01L 3/00** *(2006.01)*
**G01N 33/49** *(2006.01)* **G01N 21/82** *(2006.01)*

(52) Classification Coopérative des Brevets (CPC):
**G01N 15/00; B01L 3/502761; G01N 21/82;**
**G01N 33/4905;** B01L 2200/0647; B01L 2200/0694;
G01N 2015/0065; G01N 2015/0092

(86) Numéro de dépôt international:
**PCT/FR2016/052660**

(87) Numéro de publication internationale:
**WO 2018/069582 (19.04.2018 Gazette 2018/16)**

(54) **PROCÉDÉ DE CARACTÉRISATION D'UN ÉCHANTILLON COMPORTANT DES PARTICULES**

VERFAHREN ZUR CHARAKTERISIERUNG EINER PROBE MIT PARTIKELN

METHOD FOR CHARACTERISING A SAMPLE COMPRISING PARTICLES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**21.08.2019 Bulletin 2019/34**

(73) Titulaires:
• **Commissariat à l'Énergie Atomique
et aux Énergies Alternatives**
**75015 Paris (FR)**
• **Avalun**
**38054 Grenoble (FR)**

(72) Inventeurs:
• **HUET, Maxime**
**28700 Houville La Branche (FR)**
• **BERTHIER, Jean**
**38240 Meylan (FR)**
• **CUBIZOLLES, Myriam-Laure**
**38700 Corenc (FR)**
• **POHER, Vincent**
**62340 Guines (FR)**
• **POUTEAU, Patrick**
**38240 Meylan (FR)**
• **BUHOT, Arnaud**
**38960 St Etienne du Crossey (FR)**

• **SABATTE, Gwenola**
**38120 St Egreve (FR)**
• **SPIACZKA, Johanna**
**38650 Roissard (FR)**

(74) Mandataire: **INNOV-GROUP**
**310, avenue Berthelot**
**69372 Lyon Cedex 08 (FR)**

(56) Documents cités:
EP-A1- 2 233 923    EP-A1- 2 669 678
EP-A1- 2 975 383    US-A- 5 731 212
US-A1- 2010 035 245    US-A1- 2012 219 985
US-A1- 2015 160 244    US-A1- 2015 301 018

• **Maxime Huet ET AL: "Real time observation and
automated measurement of red blood cells
agglutination inside a passive microfluidic
biochip containing embedded reagents",
Biosensors and Bioelectronics, 20 septembre
2016 (2016-09-20), XP055386309, DOI:
10.1016/j.bios.2016.09.068 Extrait de l'Internet:
URL:http://www.sciencedirect.com/science/a
rticle/pii/S0956566316309526 [extrait le
2017-06-29]**

**Description**

## DOMAINE TECHNIQUE

L'invention est un procédé de caractérisation d'un échantillon, en particulier un échantillon biologique, comportant des particules, par une méthode optique. Le procédé est adapté à des dispositifs d'analyse portables, l'échantillon étant contenu dans une chambre fluidique compacte.

## ART ANTERIEUR

L'analyse d'échantillons biologiques à l'aide des dispositifs portables connaît un fort développement. En particulier, des dispositifs désignés par le terme anglosaxon "Point of Care", signifiant "au chevet du patient", se répandent. Ils permettent la réalisation d'analyses de façon simple et peu coûteuse. En général, l'échantillon est introduit dans une chambre fluidique, laquelle est introduite dans un dispositif d'analyse, en particulier un dispositif d'analyse optique.

Le document EP2233923 est un exemple d'application d'un dispositif optique « Point of Care » permettant une estimation de la durée de coagulation du sang. Il est de même de US2015/0301018.

Le brevet européen EP2669678 décrit un procédé permettant la détermination d'un état d'agglutination de particules, et notamment des globules rouges, en disposant un échantillon, comportant les particules, entre une source de lumière et un capteur d'image. L'échantillon est introduit dans une chambre fluidique comportant un réactif d'analyse apte à favoriser l'agglutination des globules rouges. Les globules rouges sont éclairés par la source de lumière, et génèrent, au niveau du détecteur, une image représentative de leur état d'agglutination. Plus précisément, au fur et à mesure que les globules rouges s'agglutinent, la morphologie de l'image détectée évolue. Ainsi, par une analyse d'image, il est possible de suivre l'évolution de l'agglutination des globules rouges dans l'échantillon. Il est également possible d'établir des indicateurs quantitatifs caractérisant l'état d'agglutination, c'est-à-dire la quantité de globules rouges agglutinés dans l'échantillon. En fonction du réactif d'analyse introduit, un tel dispositif permet d'effectuer un groupage sanguin, ou un dosage d'un analyte présent dans l'échantillon. Le réactif d'agglutination peut être remplacé par un réactif de coagulation, auquel cas on observe une coagulation de l'échantillon. Il en est de même de la publication Huet M, " Real time observation and automated measurement of red blood cells agglutination inside a passive microfluidic biochip containing embedded reagents", Biosensors and Bioelectronics, sept 2016. Le document US2015/160244 décrit un procédé permettant un dosage de protéines basée sur une détection d'agglutination de globules rouges.

La demande de brevet européen EP3076156 décrit un perfectionnement du procédé évoqué ci-dessus, dans lequel la source de lumière est adaptée de façon à permettre, sur chaque image acquise par le détecteur, une discrimination optimale entre des régions d'intérêt dites enrichies, dans lesquelles des particules s'agglutinent, et des régions d'intérêt dites appauvries, situées entre les régions d'intérêt enrichies.

Il est généralement admis que le réactif d'analyse doit être réparti de façon la plus homogène possible dans la chambre fluidique, de façon à ce que l'interaction avec l'échantillon soit uniforme. C'est la raison pour laquelle le réactif d'analyse est préalablement introduit dans la chambre fluidique sous forme de lyophilisat. En effet, la lyophilisation permet d'obtenir une distribution spatiale relativement homogène du réactif d'analyse dans la chambre fluidique.

Les inventeurs ont observé que des alternatives aux dispositifs ou procédés existants étaient possibles, permettant de réaliser des analyses plus précises et/ou d'améliorer la sensibilité des systèmes actuels.

## EXPOSE DE L'INVENTION

Un objet de l'invention est un procédé selon la revendication 1 annexée.

L'invention tire profit de l'établissement d'un gradient de concentration des particules et/ou d'un gradient de vitesse des particules pour positionner une région d'intérêt en fonction d'un critère de sélection. Ce dernier peut notamment être choisi parmi : une faible concentration en particules, une forte concentration en particules, une vitesse élevée des particules, une vitesse faible des particules.

Lors de l'étape a), le composé est notamment présent sous la forme d'un solide à l'état sec ou d'un gel. Lors de l'étape b), la dispersion du composé par l'échantillon peut être une dissolution lorsque le composé est soluble dans l'échantillon ou une mise en suspension lorsque le composé n'est pas soluble dans l'échantillon. L'étape g) peut comprendre le calcul d'un indicateur dépendant de l'intensité de pixels de l'image dans la région d'intérêt sélectionnée.

Selon un mode de réalisation, chambre fluidique comporte une paroi interne hydrophile, délimitée par une arête, de telle sorte que la zone de dépôt s'étend parallèlement à l'arête, et de telle sorte que lors de l'étape c), la partie appauvrie est adjacente de l'arête. Selon autre mode de réalisation, la chambre fluidique comporte une face interne sur laquelle est ménagé un motif hydrophile ou hydrophobe, de telle sorte que la zone de dépôt corresponde au motif.

Selon un mode de réalisation, un sillon est ménagé sur une face interne de la chambre fluidique, de telle sorte que la zone de dépôt corresponde au sillon.

Selon un mode de réalisation, la partie enrichie est adjacente de la partie appauvrie, la partie appauvrie s'étendant entre la partie enrichie et la zone de dépôt. Selon un mode de réalisation, la partie rapide est adjacente de la partie lente, la partie rapide s'étendant entre la partie lente et la zone de dépôt.

L'intervalle temporel de diffusion peut s'étendre entre l'introduction de l'échantillon dans la chambre fluidique et une durée de diffusion du composé dispersé dans l'échantillon, cette dernière étant supérieure à 10 secondes, voire à 30 secondes et/ou inférieure à 20 minutes.

La chambre fluidique peut comporter un réactif d'analyse apte à favoriser une agglutination des particules, ou un réactif apte à favoriser une coagulation de l'échantillon, ou un réactif apte à colorer l'échantillon, c'est-à-dire les particules et/ou le milieu liquide ou à un réactif apte à entraîner une fluorescence de l'échantillon, c'est-à-dire les particules et/ou le milieu liquide. Le réactif d'analyse peut être compris dans le composé. Lorsque le réactif d'analyse est apte à favoriser une agglutination des particules ou une coagulation de l'échantillon, la région d'intérêt sélectionnée lors de l'étape f) peut être la partie enrichie de la chambre fluidique ou la partie rapide de la chambre fluidique. Lorsque le réactif d'analyse est apte à favoriser une coloration du milieu liquide de l'échantillon, la région d'intérêt sélectionnée lors de l'étape f) peut être la partie appauvrie de l'échantillon. Lorsque le réactif d'analyse est apte à favoriser une coloration ou une fluorescence des particules de l'échantillon, la région d'intérêt sélectionnée lors de l'étape f) peut être la partie enrichie de la chambre fluidique.

Le procédé peut comporter, préalablement à l'étape a), un remplissage de la chambre fluidique par un liquide porteur, par exemple un solvant, dans lequel le composé est dispersé, par exemple en formant un soluté, puis un séchage de façon à former un dépôt sec du composé suite à l'évaporation du liquide porteur.

Le capteur d'image est configuré pour acquérir une image de la chambre fluidique. De préférence, le capteur d'image forme une image selon un plan de détection, ce dernier étant parallèle ou sensiblement parallèle au gradient du composé dispersé. Le terme sensiblement parallèle désigne parallèle à une tolérance angulaire près, cette dernière étant de préférence dans la plage angulaire $\pm$ 45°, et de préférence $\pm$ 20°. La chambre fluidique peut être interposée entre le capteur d'image et la source de lumière. Elle peut également être disposée en regard du capteur d'image et de la source de lumière. La région d'intérêt sélectionnée comporte de préférence plusieurs pixels adjacents, par exemple au moins 100 pixels, ou au moins 1000 pixels. Elle correspond à une surface d'au moins 1 mm$^2$, et généralement comprise entre 1 mm$^2$ et 20 mm$^2$. La chambre fluidique peut notamment s'étendre entre deux parois parallèles, ou sensiblement parallèles, espacées d'une distance généralement inférieure à 1 cm, voire inférieure à 0.5 cm. Dans ce cas, le plan de détection est de préférence parallèle ou sensiblement parallèle auxdites parois parallèles de la chambre fluidique. Les dimensions de la chambre fluidique peuvent être adaptées pour que l'échantillon progresse, dans cette dernière, par capillarité.

Lors de l'étape f), la région d'intérêt peut être sélectionnée manuellement par un opérateur, en fonction de l'image acquise. Elle peut également être déterminée par un algorithme de traitement d'image mis en œuvre par un processeur, sur la base d'une ou de plusieurs images acquises, en fonction du critère de sélection pris en compte. Elle peut également être déterminée suite à une phase de calibration, au cours de laquelle on forme une image d'au moins un échantillon de calibration, de façon à positionner la région d'intérêt selon le critère de sélection pris en compte. L'échantillon de calibration est de préférence considéré comme suffisamment représentatif de l'échantillon que l'on souhaite caractériser, et est introduit dans une chambre fluidique similaire à celle dans laquelle l'échantillon à caractériser est introduit. La phase de calibration permet de définir une position d'une région d'intérêt, cette dernière étant ensuite utilisée dans l'étape f).

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

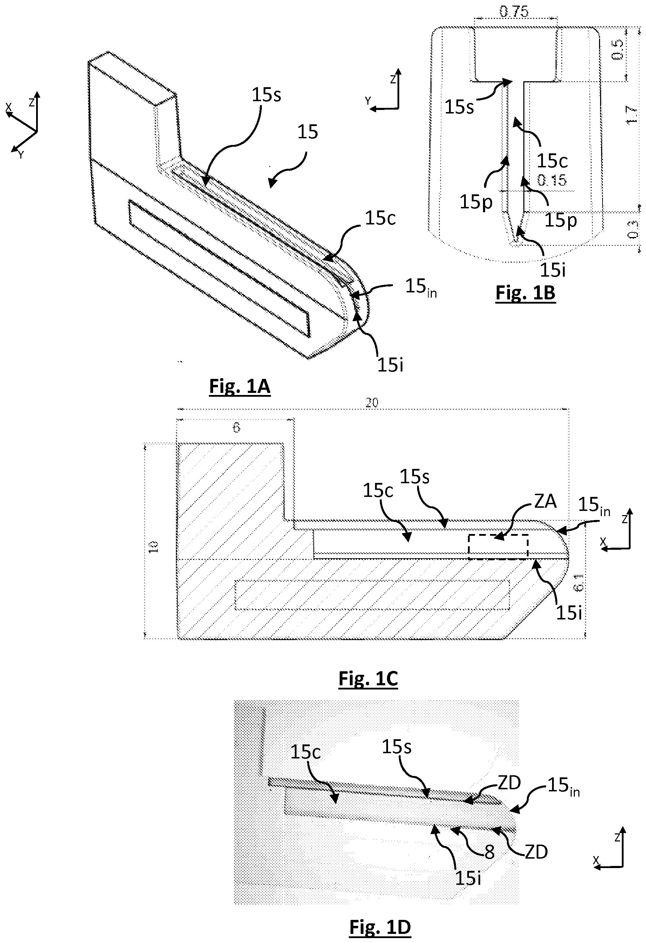

Les figures 1A, 1B et 1C représentent un composant fluidique apte à recevoir un échantillon liquide à analyser. La figure 1D est une image illustrant une répartition d'un composé sec dans le composant fluidique représenté sur les figures 1A à 1C. Les dimensions représentées sur les figures 1B et 1C sont exprimées en mm.

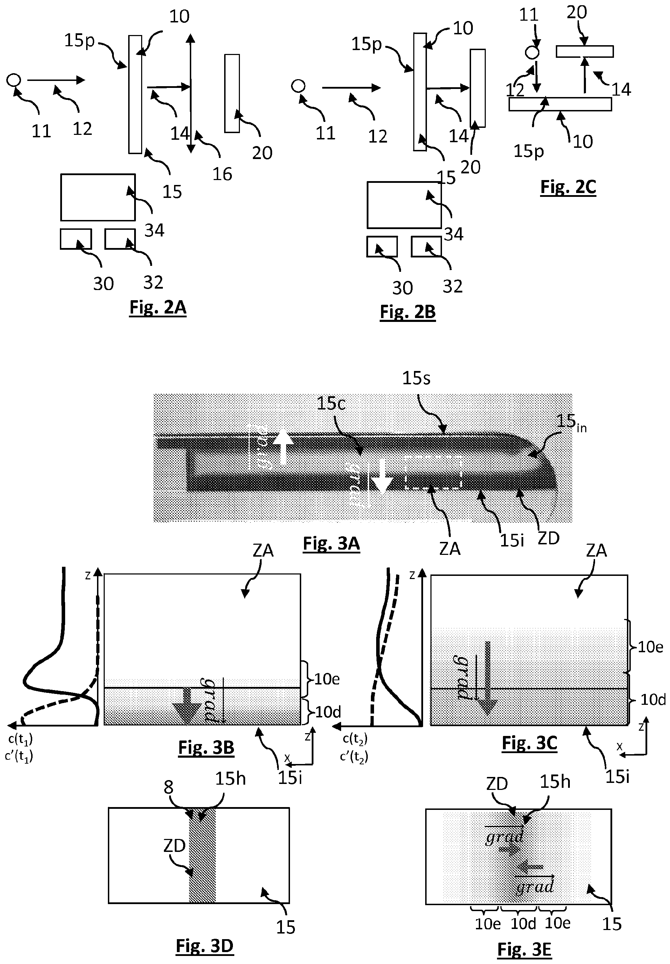

Les figures 2A, 2B et 2C représentent des modes de réalisation de dispositifs permettant la mise en œuvre de l'invention.

La figure 3A est une image illustrant une diffusion du composé sec, après dilution dans un échantillon liquide, dans lequel il forme un soluté, après introduction d'un échantillon liquide dans le composant fluidique représenté sur la figure 1D. Les figures 3B et 3C sont des schémas illustrant la répartition de particules dans le composant fluidique représenté sur la figure 3A, à deux instants suivant l'introduction d'un échantillon dans le composant fluidique.

La figure 3D illustre un deuxième exemple de chambre fluidique, sur la paroi interne de laquelle un dépôt de composé a été formé sur un motif hydrophile. La figure 3E schématise la formation d'un gradient de particules suite à une dispersion du composé par un échantillon liquide.

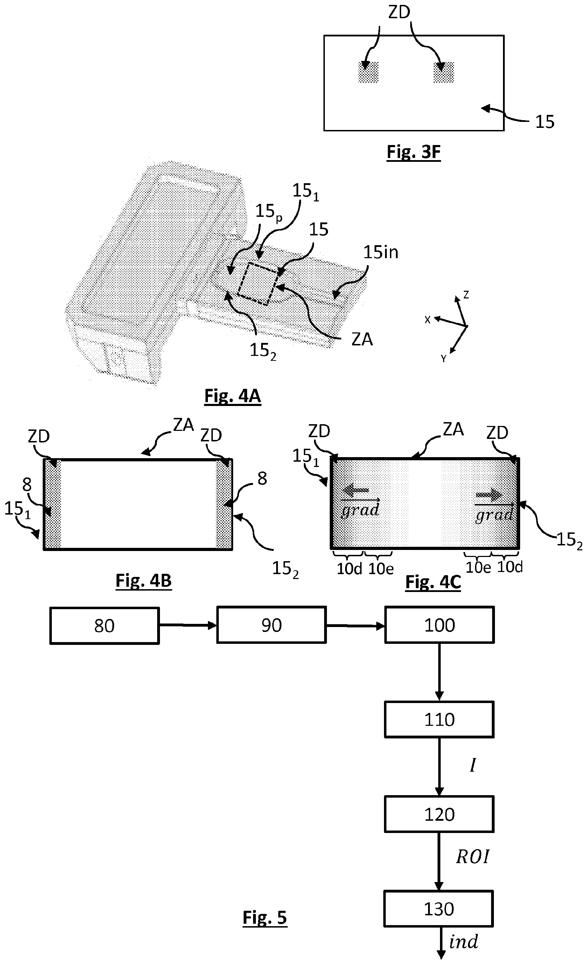

La figure 3F illustre un troisième exemple de chambre fluidique, dont la paroi interne comporte plusieurs zones de dépôts ZD distinctes les unes des autres.

La figures 4A montre un quatrième exemple de chambre fluidique. La figure 4B montre une répartition d'un composé sec dans la chambre fluidique. La figure 4C illustre la formation de gradients de diffusion du composé après introduction d'un échantillon liquide dans la chambre fluidique.

La figure 5 illustre les principales étapes d'un procédé d'analyse d'un échantillon selon l'invention.

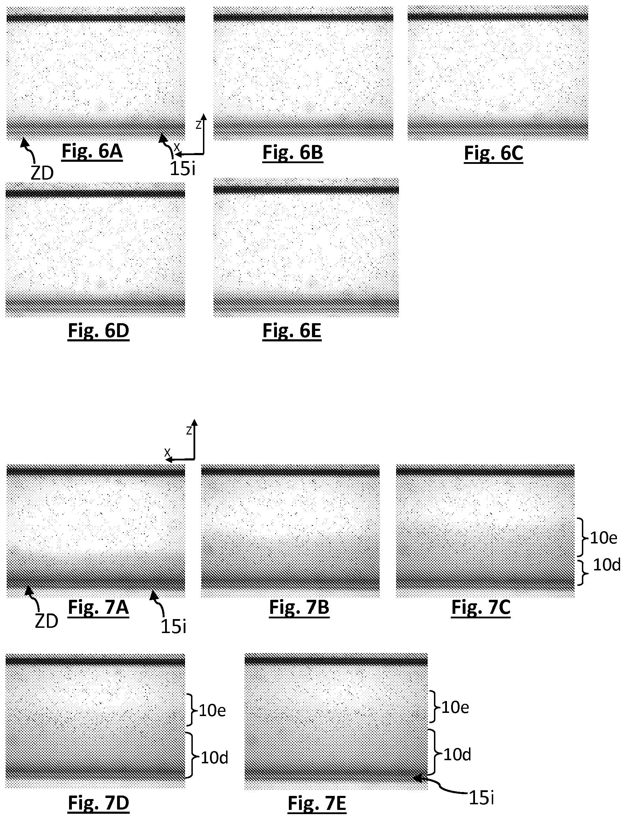

Les figures 6A, 6B, 6C, 6D et 6E représentent des images successivement acquises au cours d'un premier essai. Ces images illustrent une évolution de la répartition de microbilles dans une chambre fluidique disposée horizontalement dépourvue de composé préalablement déposé.

Les figures 7A, 7B, 7C, 7D et 7E représentent des images successivement acquises au cours d'un deuxième essai. Ces images illustrent une évolution de la répartition de microbilles dans une chambre fluidique, disposée horizontalement, comportant, au niveau de son extrémité inférieure, une zone de dépôt d'un composé.

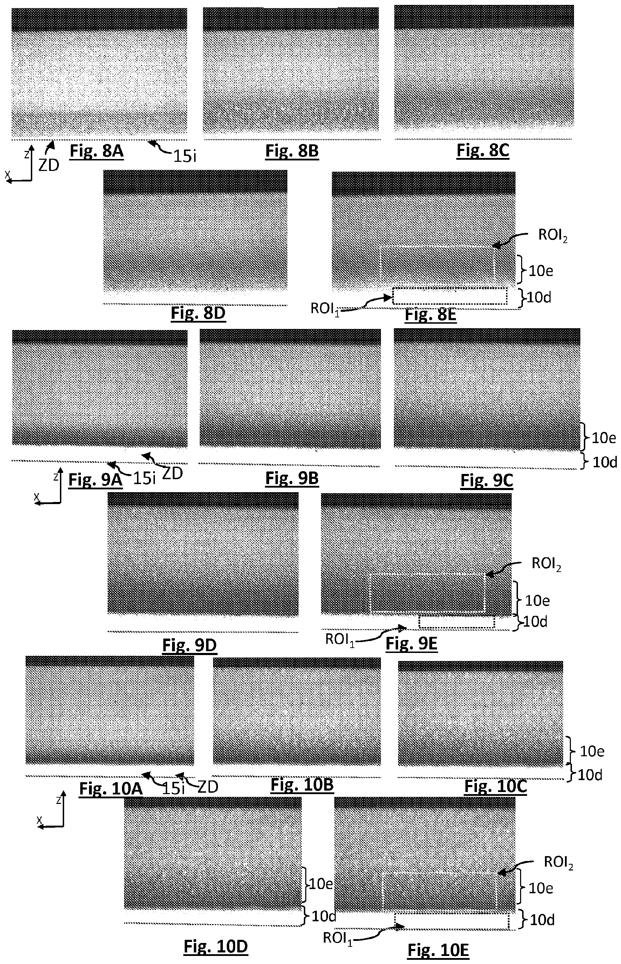

Les figures 8A, 8B, 8C, 8D et 8E représentent des images successivement acquises au cours d'un troisième essai. Ces images illustrent une évolution de la répartition de globules rouges dans une chambre fluidique, disposée horizontalement, comportant, au niveau de son extrémité inférieure, une zone de dépôt d'un composé, l'échantillon étant du sang dilué.

Les figures 9A, 9B, 9C, 9D et 9E représentent des images successivement acquises au cours d'un quatrième essai. Ces images illustrent une évolution de la répartition de globules rouges dans une chambre fluidique, disposée horizontalement, comportant, au niveau de son extrémité inférieure, une zone de dépôt d'un composé, l'échantillon étant du sang total.

Les figures 10A, 10B, 10C, 10D et 10E représentent des images successivement acquises au cours d'un cinquième essai. Ces images illustrent une évolution de la répartition de globules rouges dans une chambre fluidique, disposée horizontalement, comportant, au niveau de son extrémité inférieure, une zone de dépôt d'un composé, l'échantillon étant du sang total, les globules rouges s'agglutinant dans la chambre fluidique.

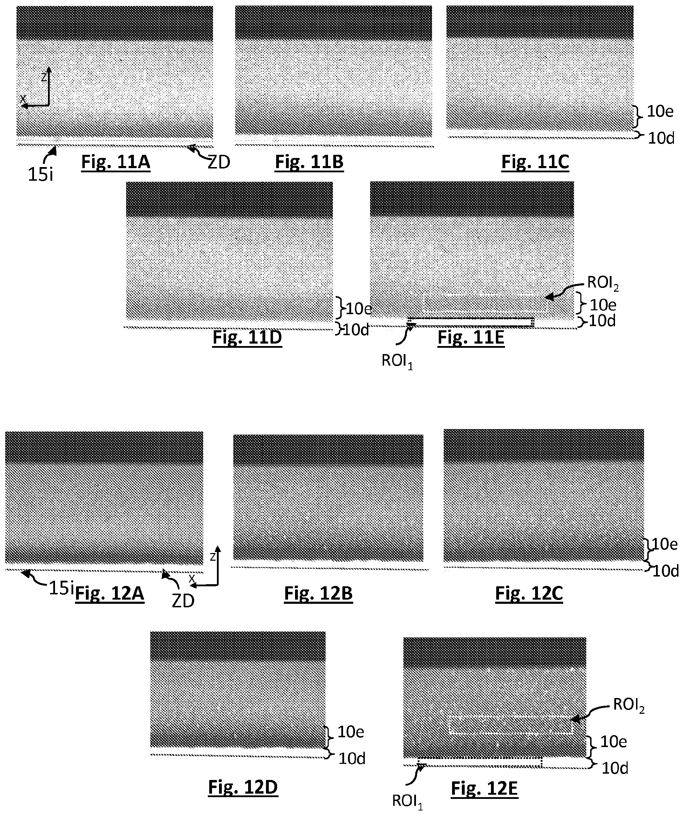

Les figures 11A, 11B, 11C, 11D et 11E représentent des images successivement acquises au cours d'un sixième essai. Ces images illustrent une évolution de la répartition de globules rouges dans une chambre fluidique, disposée verticalement, comportant, au niveau de son extrémité inférieure, une zone de dépôt d'un composé, l'échantillon étant du sang total.

Les figures 12A, 12B, 12C, 12D et 12E représentent des images successivement acquises au cours d'un septième essai. Ces images illustrent une évolution de la répartition de globules rouges dans une chambre fluidique, disposée verticalement, comportant, au niveau de son extrémité inférieure, une zone de dépôt d'un composé, l'échantillon étant du sang total, les globules rouges s'agglutinant dans la chambre fluidique.

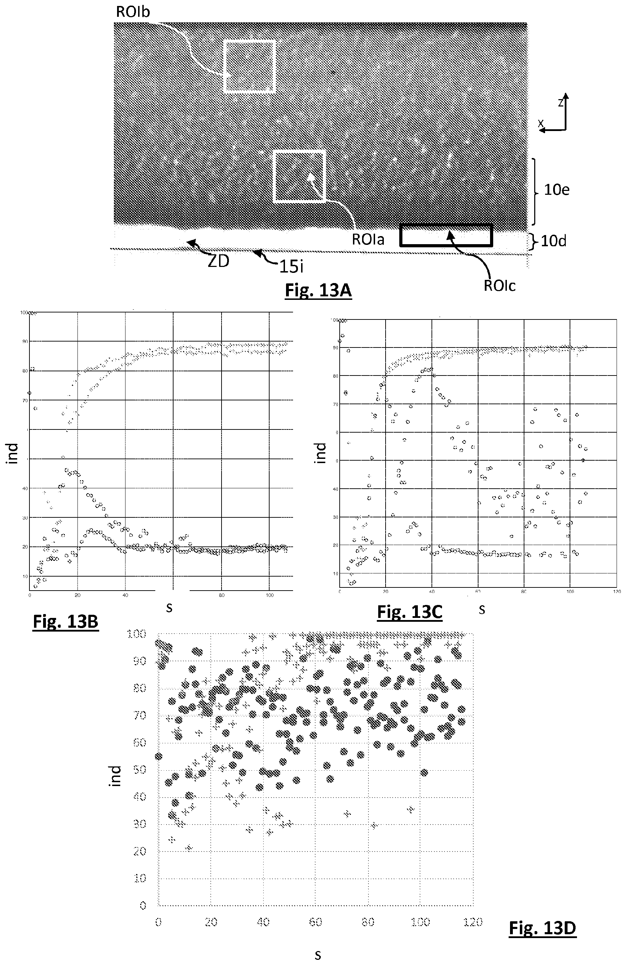

La figure 13A représente une image d'un huitième essai, au cours duquel un échantillon de sang de groupe A ou de groupe B est introduit dans une chambre fluidique disposée verticalement, telle que représentée sur les figures 1A à 1D. La figure 13B est une courbe montrant une évolution d'un indicateur de corrélation en fonction du temps, en considérant une région d'intérêt correspondant à une partie enrichie de l'échantillon, et cela pour deux échantillons positifs et deux échantillons négatifs. La figure 13C est une courbe montrant une évolution d'un indicateur de corrélation en fonction du temps, en considérant une région d'intérêt située en périphérie de la partie enrichie de l'échantillon, et cela pour deux échantillons positifs et deux échantillons négatifs. La figure 13D est une courbe montrant une évolution d'un indicateur de corrélation en fonction du temps, en considérant une région d'intérêt appauvrie de l'échantillon, et cela pour deux échantillons positifs et deux échantillons négatifs.

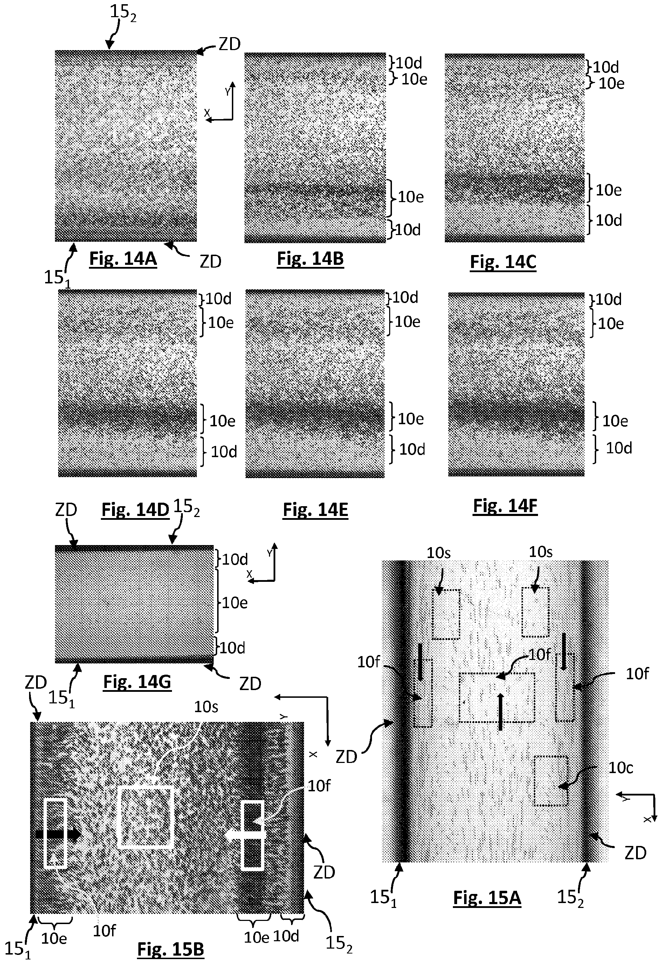

Les figures 14A, 14B, 14C, 14D, 14E et 14F représentent des images successivement acquises au cours d'un neuvième essai. Ces images illustrent une évolution de la répartition de particules dans une chambre fluidique, disposée horizontalement, telle que représentée sur la figure 4A, une zone de dépôt étant formée au niveau deux arêtes opposées de la chambre.

La figure 14G représente une image acquise au cours d'un dixième essai, cet essai étant réalisé dans des conditions similaires au neuvième essai, la concentration des particules étant plus faible.

La figure 15A illustre un onzième essai, réalisé avec une chambre fluidique telle que représentée sur la figure 4A, disposée verticalement. La figure 15B illustre un douzième essai, réalisé avec une chambre fluidique telle que représentée sur la figure 4A, disposée horizontalement. Sur les figures 15A et 15B, on a superposé des images acquises durant une période cumulée de 3 secondes. Cela permet de visualiser la vitesse des particules dans la chambre fluidique.

## EXPOSE DE MODES DE REALISATION PARTICULIERS

La figure 1A représente un composant fluidique formant une chambre fluidique 15. Un tel composant fluidique a été décrit dans la demande de brevet EP2964090. On a indiqué un repère XYZ relatif au composant. La figure 1B représente une vue en coupe de la chambre fluidique 15, selon un plan YZ. La chambre fluidique 15 est formée par un canal 15c, s'étendant, selon un axe transversal Z entre une extrémité inférieure 15i et une extrémité supérieure 15s, et selon un axe latéral Y, entre deux parois latérales parallèles 15p. La distance maximale entre les deux parois parallèles 15p est, dans cet exemple, de 150 μm. L'extrémité 15i est fermée tandis que l'extrémité supérieure 15s est ouverte. On note un rapprochement des parois latérales au niveau de l'extrémité inférieure 15i. Dans cet exemple, la hauteur selon laquelle s'étend le canal fluidique, selon l'axe Z, est de 1.5 mm, tandis que la largeur du canal, selon l'axe latéral Y, est de 0.15 mm. L'extrémité inférieure 15i est constituée par l'intersection des deux parois latérales du canal. Elle forme un fond du canal 15c s'étendant selon un axe longitudinal X. Les parois latérales font préférablement l'objet d'un traitement préalable, de façon à être hydrophiles. Par matériau hydrophile, on entend un matériau tel que l'angle de contact formé par une goutte sur le matériau, au niveau de la ligne triple, est inférieur à 90°. Une telle géométrie est propre à un écoulement capillaire spontané d'un échantillon liquide dans le canal 15c. Ainsi, comme décrit dans la demande de brevet EP2964090, un échantillon liquide est apte à pénétrer dans la chambre fluidique 15 par une fente d'entrée 15$_{in}$ visible sur les figures 1A et 1C, puis à progresser spontanément selon l'axe longitudinal X, en étant entraîné au niveau de l'extrémité inférieure 15i.

La figure 1C représente une vue en coupe du composant fluidique, selon un plan XZ, passant par l'extrémité inférieure 15i, formant le fond du canal 15c. Les parties hachurées sont pleines, tandis que l'échantillon liquide est se propage dans les parties claires du composant, définissant la chambre fluidique 15, en étant tiré le long du fond du canal formé par l'extrémité inférieure 15i. Il est alors possible d'acquérir une image de l'échantillon dans une zone d'analyse ZA du canal 15c, une telle zone d'analyse étant représentée par un cadre en pointillés sur la figure 1C. Pour cela, le composant fluidique peut être disposé entre une source de lumière et un capteur d'image, comme représenté sur les figures 2A et 2B.

Comme décrit en lien avec l'art antérieur, un tel composant fluidique peut être utilisé pour l'analyse d'un échantillon 10 comportant du sang, et de façon plus générale d'un échantillon comportant des particules. Par particules, on entend par exemple des cellules, et notamment des cellules sanguines, mais il peut également s'agir de microorganismes, des virus, des spores, ou des microbilles, par exemple des microbilles magnétiques, usuellement mises en œuvre dans des applications biologiques, ou encore des microalgues. Il peut également s'agir de vésicules, de liposomes ou de gouttelettes insolubles dans le milieu liquide, par exemple des gouttelettes d'huile dispersées dans une phase aqueuse.

De préférence, les particules ont un diamètre, ou sont inscrites dans un diamètre, inférieur à 100 μm, et de préférence inférieur à 50 μm ou 20 μm. Les particules baignent dans un milieu liquide, ce dernier étant du sérum ou du plasma, éventuellement dilué, lorsque l'échantillon est du sang. Le milieu liquide peut être un autre liquide, notamment un liquide corporel, un liquide prélevé dans l'environnement, ou un liquide résultant d'un procédé industriel.

Les inventeurs estiment qu'il est préférable que le diamètre des particules, ou dans lequel est inscrit chaque particule, soit supérieur à 500 nm, voire à 1 μm.

La concentration des particules peut être comprise, en volume, entre 0.1% et 50%. Une telle plage a été testée avec succès sur des particules dont le diamètre est de 10 μm.

A l'intérieur de la chambre fluidique 15, on peut disposer des réactifs. Il peut par exemple s'agir de réactifs propices au déclenchement d'une réaction de coagulation de l'échantillon, ou au déclenchement d'une réaction d'agglutinations de particules. De tels réactifs ont été décrits dans les demandes EP2669678 ou EP3076156. Il peut notamment s'agir de protéines, d'anticorps ou d'enzymes. Selon l'invention, les réactifs sont préalablement embarqués, au sens où ils sont présents dans la chambre fluidique 15 avant l'introduction de l'échantillon 10 dans cette dernière. Lorsque l'échantillon 10 pénètre dans la chambre fluidique 15, un mélange se forme de façon à obtenir une réaction à l'intérieur de l'échantillon, qu'il s'agisse d'une coagulation, d'une agglutination, ou encore d'une variation des propriétés optiques de l'échantillon, par exemple une coloration ou une fluorescence. Une telle réaction peut être observée par un capteur d'image optiquement couplé à la zone d'analyse ZA. Des procédés de traitement d'image peuvent alors être mis en œuvre, de façon à caractériser l'échantillon. Par caractériser l'échantillon, on entend par exemple déterminer un paramètre de coagulation, ou un niveau d'agglutination de particules, ou l'apparition d'une fluorescence ou d'une coloration. La caractérisation peut également permettre le dosage d'un analyte.

La figure 2A illustre un dispositif d'analyse d'un échantillon liquide pouvant être utilisé pour mettre en œuvre un procédé selon l'invention. Une source de lumière 11 émet une onde lumineuse incidente 12, se propageant jusqu'à l'échantillon 10 disposé dans une chambre fluidique 15. La source de lumière 11 peut être une diode électroluminescente, une source laser, ou une source de lumière blanche. Un filtre peut être disposé entre la source de lumière 11 et la chambre fluidique 15, de façon à délimiter une bande spectrale de l'onde lumineuse incidente 12.

La chambre fluidique 15 peut être telle que décrite en lien avec les figures 1A à 1C. Sous l'effet de son illumination par l'onde lumineuse incidente 11, l'échantillon 10 transmet une onde lumineuse dite onde transmise 14, cette dernière se propageant jusqu'à un capteur d'image 20 à travers un système optique de focalisation 16. Le système optique de focalisation permet un couplage optique du capteur d'image 20 avec la zone d'analyse ZA de la chambre fluidique 15, telle que précédemment décrite. Le capteur d'image 20 comporte généralement des pixels arrangés selon une matrice, s'étendant selon un plan de détection. Il peut notamment s'agir d'un capteur CMOS ou CCD. Chaque image *I* acquise par le capteur d'image 20 est transmise à un processeur 30, par exemple un microprocesseur. Ce microprocesseur est relié à une mémoire 32 dans laquelle sont stockées des instructions permettant un traitement d'image approprié. Le processeur 30 peut être relié à un écran 34.

Le système optique de focalisation 16 est optionnel. Ainsi, comme représenté sur la figure 2B, le capteur d'image 20 peut être disposé à une faible distance de la chambre fluidique 15, sans optique de grossissement disposé entre le capteur d'image et la chambre fluidique. Cela n'exclut pas la présence de microlentilles de focalisation au niveau des pixels du capteur d'image 20.

Selon un autre mode de réalisation, représenté sur la figure 2C, la source de lumière 11 et le capteur d'image 20 sont disposés en regard de la même face 15p de la chambre fluidique 15. Le capteur d'image collecte une onde 14 transmise par l'échantillon après réflexion ou rétrodiffusion par l'échantillon. L'échantillon peut éventuellement avoir été disposé sur un support réfléchissant. L'onde 14 atteignant le détecteur 20 peut être une onde de fluorescence émise par l'échantillon. Il s'agit d'une configuration dite en réflexion, les figures 2A et 2B représentant des configurations dites en transmission.

La chambre fluidique 15 comporte un composé 8 préalablement déposé selon une zone de dépôt ZD s'étendant à l'intérieur de la chambre fluidique 15. Le composé 8 est destiné à être dispersé, et par exemple solubilisé, par l'échantillon liquide que l'on souhaite caractériser. Le terme composé désigne des molécules d'un même type ou un mélange de molécules. Le composé est présent, dans la chambre fluidique, sous une forme solide ou sous la forme d'un gel. La zone de dépôt, selon laquelle s'étend le composé, est délimitée. Par délimitée, on entend qu'elle ne recouvre pas toutes les parois internes de la chambre fluidique, ou tout le volume interne défini par la chambre fluidique, mais s'étend dans la chambre fluidique en définissant un motif délimité. Ainsi, la zone de dépôt peut être une ligne ou une forme délimitée quelconque, définissant un motif recouvrant partiellement les parois internes de la chambre fluidique. Le composé 8 peut être soluble dans l'échantillon liquide à analyser, dans lequel il forme un soluté. Le composé 8 peut avoir été introduit dans la chambre fluidique 15 en ayant été dispersé dans un liquide dit liquide porteur. Le composé 8 peut par exemple être soluble dans le liquide porteur, ce dernier étant alors un solvant dans lequel le composé 8 forme un soluté. Au cours d'une phase de préparation, le liquide porteur est introduit dans la chambre fluidique. La chambre fluidique subit alors une phase de séchage, au cours de laquelle le liquide porteur s'évapore, formant un résidu sec à l'intérieur de la chambre fluidique, ce résidu formant le composé 8. Le composé 8 peut comprendre des réactifs aptes au déclenchement de réactions d'agglutination ou de coagulation, tels que précédemment décrits. En complément ou de façon alternative, le composé 8 peut comprendre un agent colorant, ou un agent fluorescent. Dans d'autres modes de réalisation, le composé 8 ne contient pas de tels réactifs. Il peut par exemple s'agir d'un polymère soluble (par exemple un polyéthylène glycol, du Dextran), d'un sucre, d'un sel, n'ayant pour seule fonction que la formation d'un gradient concentration de composé comme décrit ultérieurement.

Lorsque les parois délimitant la chambre fluidique 15 sont hydrophiles, lors de la phase de séchage, le liquide porteur tend à s'accumuler le long des arêtes délimitant la chambre fluidique, en formant, par capillarité, un doigt liquide au niveau des arêtes. A la fin du séchage, le composé 8 est essentiellement déposé le long des arêtes, sous une forme déshydratée. Des exemples de telles configurations seront donnés ci-après. Alternativement, on peut former, sur une paroi interne de la chambre fluidique, un motif hydrophile 15h, de telle sorte qu'au cours de la phase de séchage, le composé 8 se dépose essentiellement selon le motif hydrophile. Ainsi, d'une façon générale, la chambre fluidique comporte un composé 8, destiné à être dispersé par l'échantillon 10. Préalablement à l'introduction de l'échantillon, le composé 8 est présent dans la chambre fluidique 15 et s'étend selon une zone de dépôt ZD délimitée.

La chambre fluidique peut comporter plusieurs zones de dépôt ZD espacées les unes des autres, par exemple sous la forme de plots, comme représenté sur la figure 3F. Dans ce cas, les composés respectivement déposés selon les différentes zones de dépôts peuvent être différents les uns des autres.

Comme précédemment indiqué, le composé 8 est apte à être dispersé dans l'échantillon 10, en étant par exemple soluble dans ce dernier. Ainsi, lorsque l'échantillon 10 pénètre dans la chambre fluidique 15, il disperse peu à peu le composé 8, ce qui entraîne une diffusion du composé 8 dans la chambre fluidique 15. La diffusion a tout d'abord lieu au voisinage de la zone de dépôt ZD, et gagne progressivement toute ou partie de la chambre fluidique 15. Ainsi, durant un intervalle temporel $\Delta t$, dit de diffusion, pouvant durer quelques secondes, voire quelques minutes, par exemple 10 minutes, un gradient de concentration du composé $\overrightarrow{grad}$ se forme dans la chambre fluidique 15, notamment au voisinage de la zone de dépôt ZD. Le gradient concentration de composé $\overrightarrow{grad}$ est généralement maximal au voisinage de la zone de dépôt, et est orienté selon une direction orthogonale à une direction selon laquelle s'étend la zone de dépôt ZD. L'intervalle temporel $\Delta t$ dépend de la dispersion et de la diffusion du composé déposé sur la zone de dépôt ZD. Il est généralement supérieur à 10 secondes et généralement inférieur à 20 minutes.

Au cours d'essais expérimentaux reportés ci-après, les inventeurs ont remarqué que le gradient de concentration du composé $\overrightarrow{grad}$ a un effet sur la répartition des particules de l'échantillon, à l'intérieur de la chambre fluidique 15. Le gradient de composé dispersé peut également avoir un effet sur la vitesse des particules dans la chambre fluidique 15.

L'apparition de gradients de particules, sous l'effet de gradients de solutés, a déjà été reportée dans la littérature.

Par exemple, les effets de la diffusiophorèse dans des colloïdes ont été décrits dans la publication suivante : Anderson John, "Diffusiophoresis : Migration of Colloidal Particles in Gradients of Solute Concentration", Separation and Purification Reviews, 13:1 67-103. La diffusion de macromolécules dans un gradient de molécules a également été décrite dans Abécassis B, "Osmotic manipulation of particles for microfluidic applications" ; New J. Phys 11, 075022.

Un élément clef de l'invention est d'utiliser l'apparition du gradient $\overrightarrow{grad}$ du composé 8 pour optimiser une caractérisation de l'échantillon 10 par une analyse d'image. En effet, les inventeurs ont montré que le gradient $\overrightarrow{grad}$ de concentration du composé peut permettre la formation d'une partie 10e dite enrichie, riche en particules, et d'une partie 10d dite appauvrie, dans laquelle la concentration de particules est moindre que dans la partie enrichie. En fonction du type de caractérisation, une image peut être acquise, lors de l'apparition du gradient $\overrightarrow{grad}$ de composé, dans une région d'intérêt de la chambre fluidique 15 correspondant soit à la partie enrichie 10e, soit à la partie appauvrie 10d.

Les inventeurs ont également montré que le gradient $\overrightarrow{grad}$ de concentration du composé 8 dispersé peut permettre la formation d'une partie 10f, dite rapide, dans laquelle les particules ont une vitesse plus élevée que la vitesse moyenne des particules dans la chambre fluidique. Le gradient de concentration $\overrightarrow{grad}$ du composé 8 peut également permettre la formation d'une partie 10s, dite lente, dans laquelle les particules ont une vitesse plus lente que la vitesse moyenne des particules. Une image I peut alors être acquise, lors de l'apparition du gradient $\overrightarrow{grad}$ de concentration du composé 8, dans une région d'intérêt de la chambre fluidique correspondant soit à une partie rapide 10f, soit à une partie lente 10s. En outre, il a été observé qu'à l'interface entre une partie lente 10s et une partie rapide 10f, les particules peuvent se déplacer selon des directions contraires, les particules de la partie rapide 10f se déplaçant dans un sens, tandis que les particules de la partie lente 10s se déplacent selon un sens opposé. L'interface entre les deux parties définit une partie de cisaillement 10c, dans laquelle les particules peuvent subir une contrainte de cisaillement.

Il est préférable que le gradient $\overrightarrow{grad}$ de concentration du composé 8 s'étende parallèlement au plan de détection défini par le capteur d'image, ou sensiblement parallèlement à ce dernier. Cela permet de mieux distinguer, sur l'image acquise, une partie enrichie ou appauvrie ou lente ou rapide. Il est préférable que le gradient de concentration ne soit pas perpendiculaire au plan de détection.

La figure 1D représente une image d'un composant fluidique tel que représenté sur les figures 1A à 1C. La chambre fluidique 15 a fait l'objet d'un dépôt préalable d'un composé 8, à l'état sec, de couleur orange, se concentrant essentiellement au niveau des arêtes. La composition du composé sera décrite ci-après en lien avec les essais expérimentaux. Les parois internes de la chambre fluidique 15 sont hydrophiles. Le composé 8 est introduit dans la chambre fluidique 15 en étant dispersé dans un liquide porteur. Lors d'une phase de séchage, le liquide porteur s'évapore et le composé tend à se déposer, par capillarité, au niveau des arêtes de la chambre fluidique 15, du fait de leur mouillabilité élevée. Sur la figure 1D, le dépôt du composé 8 apparaît sous la forme de lignes sombres s'étendant parallèlement aux arêtes de la chambre. Ainsi, chaque arête, et notamment l'arête formée au niveau de l'extrémité inférieure 15i, forme une zone de dépôt ZD.

La figure 3A représente une image de la chambre fluidique telle que représentée sur la figure 1D, après introduction d'un échantillon liquide transparent. Dans cet exemple, l'échantillon introduit est du plasma sanguin. Le composé sec 8 est dispersé, en étant solubilisé par le plasma sanguin, et diffuse progressivement dans la chambre fluidique 15. Le composé comporte un agent fluorescent, permettant de suivre optiquement sa diffusion. L'image de la figure 3A a été formée dans une bande spectrale verte, dans laquelle se situe la longueur d'onde de fluorescence de l'agent fluorescent. La diffusion du composé dispersé apparaît selon des bandes sombres apparaissant au voisinage des arêtes, et en particulier de l'extrémité inférieure 15i. On a représenté, sur la figure 3A, les gradients $\overrightarrow{grad}$ de composé dispersé se formant suite à la dispersion et à la diffusion du composé dans le milieu liquide.

Les inventeurs ont constaté que le gradient $\overrightarrow{grad}$ de composé dispersé a une influence sur la répartition des particules dans la chambre fluidique 15. Les figures 3B et 3C schématisent la formation d'un tel gradient dans la zone d'analyse ZA, au voisinage de l'extrémité inférieure 15i de la chambre fluidique 15, à deux instants différents. Un premier instant $t_1$, représenté sur la figure 3B, correspond au début de la dispersion et de la diffusion du composé 8 par l'échantillon 10 pénétrant dans la chambre fluidique 15. Par exemple, ce premier instant $t_1$ correspond à une durée de 5 secondes après l'introduction de l'échantillon 10. La concentration de composé est maximale au niveau de l'extrémité inférieure 15i et décroît rapidement au fur et à mesure que l'on s'en éloigne, selon l'axe transversal Z. Cette concentration est représentée par des niveaux de gris sur la figure 3B, ainsi que sur le profil en pointillés représentant la concentration du composé $c(t_1)$ en fonction de la distance z par rapport à l'extrémité inférieure 15i. Le gradient $\overrightarrow{grad}$ de concentration du composé 8 est maximal au voisinage de l'extrémité inférieure 15i. Un tel gradient a une influence sur la répartition des particules dans la chambre fluidique 15, cette répartition étant schématisée par un profil de la concentration en particules $c'(t_1)$ en fonction de la distance z par rapport à l'extrémité inférieure 15i, ce profil étant représenté par un trait plein sur la figure 3B. Au voisinage de l'extrémité inférieure 15i, la concentration en particules est faible, voire nulle. Cela s'explique par le fait qu'au voisinage de cette extrémité, la concentration en composé c(t1) est importante, ce qui repousse les particules. Ainsi, dans la chambre fluidique 15, une partie appauvrie, ou partie déplétée 10d se forme. Le terme partie appauvrie, ou zone appauvrie, désigne une région dans laquelle la concentration en particules est inférieure à une concentration moyenne en particules dans l'échantillon 10.

Les particules repoussées de l'extrémité inférieure 15i s'accumulent au voisinage de la partie appauvrie 10d. Sur le profil c'($t_1$)cela correspond à un pic correspondant à une partie de la chambre fluidique, dite partie enrichie 10e, ou zone enrichie 10e, dans laquelle la concentration en particules est supérieure à la concentration moyenne en particules dans l'échantillon 10.

La figure 3C représente une distribution spatiale du composé et des particules à un deuxième instant $t_2$, postérieur au premier instant $t_1$. Le deuxième instant correspond par exemple à une durée de 1 minute après l'introduction de l'échantillon 10 dans la chambre fluidique 15. La concentration c(t2) du composé 8 est toujours maximale au niveau de l'extrémité inférieure 15i, mais tend à s'homogénéiser. De ce fait, le profil de concentration en composé 8 en fonction de la distance z par rapport à l'extrémité inférieure est moins marqué. Le gradient de composé $\overrightarrow{grad}$ s'atténue. Par conséquent, il subsiste une partie appauvrie 10d au voisinage de l'extrémité inférieure 15i, et une partie enrichie 10e s'étendant de façon adjacente de la région appauvrie 10d. La différence de concentration en particules, entre ces deux parties, tend à s'atténuer, comme le montre le profil c'($t_2$) en fonction de l'axe Z. Quelques minutes après l'introduction de l'échantillon dans l'échantillon, la répartition du composé 8 dispersé dans la chambre fluidique tend à s'homogénéiser, de même que la répartition des particules dans la chambre fluidique 15.

L'apparition d'une partie enrichie et d'une partie appauvrie dans une chambre fluidique, telle que schématisée sur les figures 3A à 3C, est commentée plus en détail dans la description d'essais expérimentaux qui suit.

Les figures 3D et 3E représentent un autre exemple de formation d'un gradient $\overrightarrow{grad}$ de concentration de composé dans un deuxième exemple de chambre fluidique, entraînant, temporairement, l'apparition d'une partie appauvrie 10d et d'une partie enrichie 10e dans une chambre fluidique 15. Selon cet exemple, une paroi de la chambre fluidique comporte un motif hydrophile 15h. Un tel motif peut être réalisé en appliquant un traitement local à la paroi interne de la chambre fluidique, de façon à former un motif hydrophile prédéterminé. Par motif hydrophile, on entend une partie hydrophile de forme prédéfinie. Dans cet exemple, le motif hydrophile prend la forme d'une bande s'étendant à travers la paroi interne. De la même manière que dans l'exemple précédent, la chambre fluidique 15 peut être remplie par un liquide porteur dans lequel le composé 8 est dissout. Au cours du séchage du liquide porteur, le composé 8 se dépose, à l'état sec, essentiellement sur le motif hydrophile 15h, formant une zone de dépôt ZD. La figure 3D illustre la paroi de la chambre fluidique, après formation du composé sec réparti selon la bande hydrophile 15h. Lors de l'introduction d'un échantillon liquide 10, comportant des particules, dans la chambre fluidique, un gradient de concentration composé dispersé apparaît au voisinage du motif hydrophile 15h, comme représenté sur la figure 3E. De ce fait, de façon analogue à la configuration représentée sur les figures 3B et 3C, une partie appauvrie 10d et une partie enrichie 10e se forment dans la chambre fluidique 15. Plus précisément, la partie appauvrie 10d se forme de part et d'autre de la zone de dépôt ZD du composé 8, une partie enrichie 10e se formant de part et d'autre de la partie appauvrie 10d.

Selon un autre mode de réalisation, le composé est déposé en étant mélangé à un liquide, une ou plusieurs gouttes du liquide étant déposées sur une paroi interne hydrophobe d'une chambre fluidique. Sous l'effet de l'évaporation du liquide, une zone de dépôt ZD se forme à l'endroit où ont été déposées les gouttes.

Selon un autre mode de réalisation, un sillon est formé dans une paroi interne de la chambre fluidique. Le composé est déposé dans le sillon en étant mélangé à un liquide. Une ou plusieurs gouttes du liquide sont déposées dans le sillon, ce dernier permettant de définir une position du liquide dans la chambre fluidique. Sous l'effet de l'évaporation du liquide, une zone de dépôt ZD se forme au niveau du sillon.

La figure 4A représente un autre exemple de composant fluidique, ce composant étant décrit dans la demande de brevet WO2016139409. Un canal d'amenée d'un échantillon liquide, s'étend, selon un axe longitudinal X, entre une chambre fluidique 15 et une entrée $15_{in}$ par laquelle l'échantillon est introduit. La chambre fluidique 15 comporte deux parois principales $15_p$ parallèles à un plan XY, et reliées par deux parois latérales $15_1$ et $15_2$ s'étendant parallèlement à un plan XZ. La distance entre les deux parois principales $15_p$ peut être comprise entre $50\mu m$ et 1 mm. On a représenté une zone d'analyse ZA, s'étendant au travers de la chambre fluidique, destinée à une analyse de l'échantillon lorsque le composant fluidique est interposé entre un capteur d'image 20 et une source de lumière 11, comme représenté sur les figures 2A à 2C. Les parois latérales $15_1$ et $15_2$ forment des arêtes avec chaque paroi principale 15p. Les parois principales sont hydrophiles. Préalablement à son utilisation, le composant fluidique a fait l'objet d'un dépôt d'un composé sec 8. De la même manière que dans les exemples précédents, le composé 8 est introduit en étant dissout dans un liquide porteur. Lors du séchage du liquide porteur, le composé tend à se concentrer spontanément au niveau des arêtes, au niveau desquelles un doigt liquide se forme, par capillarité. Lors de l'évaporation du liquide porteur, le composé 8 subsiste à l'état sec au niveau des arêtes, formant un dépôt localisé selon une zone de dépôt ZD s'étendant le long des arêtes, entre chaque paroi latérale et les parois principales 15p.

La figure 4B schématise la zone d'analyse ZA, bordée par une zone de dépôt ZD de composé sec 8 au niveau de chaque paroi latérale $15_1$ et $15_2$, chaque zone de dépôt s'étendant parallèlement aux arêtes entre les parois latérales et les faces principales 15p. Lors de l'introduction d'un échantillon liquide 10, le composé 8 se dissout peu à peu dans l'échantillon et forme des gradients $\overrightarrow{grad}$ de concentration tels que représentés sur la figure 4C. Ces gradients $\overrightarrow{grad}$ sont

représentés par des niveaux de gris, représentant la concentration du composé, ainsi que par des flèches. De même que dans les exemples représentés sur les figures 3B, 3C et 3E, une partie appauvrie 10d se forme de façon adjacente à chaque zone de dépôt ZD du composé. Une partie enrichie 10e se forme également en bordure de chaque partie appauvrie 10d.

Un élément important de l'invention est l'obtention, à l'aide d'un capteur d'image 20, d'une image *I* d'une zone d'analyse ZA d'une chambre fluidique dans laquelle, suite à la formation du gradient de concentration de composé, une partie enrichie et/ou une partie appauvrie (et/ou une partie rapide et/ou une partie lente) se forment. En sélectionnant, sur l'image *I*, une région d'intérêt ROI correspondant à une partie enrichie 10e, ou à une partie appauvrie 10d, ou à une partie rapide 10f ou à une partie lente 10s, on peut obtenir une caractérisation de l'échantillon 10. La caractérisation de l'échantillon peut être une détection d'une propriété optique du milieu liquide dans lequel baignent les particules, par exemple un changement de couleur ou une fluorescence ; dans ce cas la région d'intérêt sélectionnée sur l'image correspond de préférence à une partie appauvrie 10. La caractérisation de l'échantillon peut être une détection d'une coagulation de l'échantillon, ou d'une agglomération des particules; dans ce cas, la région d'intérêt sélectionnée sur l'image correspond de préférence à une partie enrichie 10e, ou à une partie rapide 10f.

La figure 5 représente les principales étapes d'un procédé de caractérisation d'un échantillon selon l'invention :

Etape 100 : introduction de l'échantillon dans la chambre fluidique 15, ce qui entraîne une diffusion progressive du composé 8 dans la chambre fluidique, entraînant la formation d'une partie enrichie 10e et/ou d'une partie appauvrie 10d, ou d'une partie rapide 10f et/ou d'une partie lente 10s dans la chambre fluidique.

Etape 110 : illumination de la chambre fluidique 15 à l'aide d'une source de lumière 11 et acquisition d'une image de la chambre fluidique, à l'aide d'un capteur d'image 20. L'image acquise *I* correspond généralement à une zone d'analyse ZA de la chambre fluidique, définie par le champ d'observation du capteur d'image 20.

Etape 120 : sélection d'une région d'intérêt ROI dans l'image acquise, cette région d'intérêt correspondant à une partie appauvrie 10d, ou à une partie enrichie 10e, ou à une partie rapide 10f ou à une partie lente 10s. La sélection peut être effectuée manuellement par un opérateur, par exemple sur la base d'un affichage de l'image acquise sur l'écran 34. Elle peut également être réalisée automatiquement, selon un algorithme de sélection programmé dans la mémoire 32 et mis en œuvre par le processeur 30. Il peut par exemple s'agir d'un algorithme de type seuillage selon un niveau de gris, ou d'une sélection selon une région d'intérêt prédéfinie à partir d'essais de calibration effectués préalablement.

Etape 130 : caractérisation de l'échantillon à partir de la région d'intérêt ROI sélectionnée. Il s'agit généralement de quantifier un indicateur *ind* caractérisant l'image dans la région d'intérêt sélectionnée. L'indicateur de caractérisation *ind* dépend de l'intensité des pixels dans la région d'intérêt sélectionnée. Il peut s'agir d'un indicateur statistique relatif à une distribution de l'intensité des pixels dans la région d'intérêt sélectionnée, par exemple une moyenne, une médiane, un niveau minimal, maximal, un indicateur de dispersion ou un autre moment de la distribution. Il peut également s'agir d'un nombre de pixels dans une plage d'intensité prédéterminée. L'indicateur peut être un indicateur de corrélation spatiale entre les régions d'intérêt respectivement sélectionnées dans deux images acquises à des instants différents. De tels indicateurs sont reportés dans le brevet européen EP2669678. L'indicateur peut être calculé à partir de l'intensité des pixels de la région d'intérêt, par un algorithme programmé dans la mémoire 32, et mis en œuvre par le processeur 30.

Préalablement à ces étapes, une préparation de la chambre fluidique 15 peut être effectuée, selon les étapes 80 et 90.

Etape 80 : remplissage de la chambre fluidique 15 par un liquide, dit liquide porteur dans lequel un composé 8 est dispersé, en étant par exemple solubilisé.

Etape 90 : séchage. Au cours du séchage, le liquide porteur forme, par capillarité, un doigt fluidique, par exemple au niveau d'une arête, ou au niveau d'un motif hydrophile ou hydrophobe préalablement délimité. Il s'évapore peu à peu, de telle sorte qu'un dépôt de composé 8 sec se forme, par déshydratation, selon une zone de dépôt ZD délimitée. Ainsi, le dépôt de composé n'est pas homogène dans la chambre fluidique 15, ce qui est propice à la formation d'un gradient $\overrightarrow{grad}$ de concentration du composé 8 lors de sa dispersion par l'échantillon liquide 10. La zone de dépôt ZD se forme au niveau des parties de la chambre fluidique dans lesquelles la mouillabilité est maximale. Le séchage peut être effectué à l'air libre ou dans un dessiccateur, éventuellement à l'aide d'une pompe à vide.

En fonction de la caractérisation souhaitée, le composé 8 peut comporter un réactif propice à l'établissement d'une réaction dans l'échantillon. Il peut s'agir d'un indicateur coloré, d'un marqueur fluorescent, d'un agent agglutinant, susceptible d'engendrer une agglutination de particules ou d'un agent coagulant, susceptible d'engendrer une coagulation de l'échantillon. Alternativement, la seule fonction du composé est de se disperser dans l'échantillon, de façon à former un gradient de concentration du composé. Dans ce cas, comme précédemment décrit, le composé peut être un sucre, un polymère ou un sel.

Essais expérimentaux.

Dans les essais expérimentaux présentés ci-après, on va étudier une mise en œuvre de l'invention appliquée à l'agglutination de globules rouges dans un échantillon de sang total ou de sang dilué. On a utilisé le composant fluidique décrit en lien avec les figures 1A à 1D, la zone d'analyse ZA étant limitrophe de l'extrémité inférieure 15i, comme cela est représenté sur la figure 1C. Dans certains essais, le composant a fait l'objet d'un remplissage par une solution de préparation, faisant office de liquide porteur, comportant :

- 1/5 en volume d'un réactif anti-A du kit de dosage sanguin Diagast - Groupakit, formant un réactif agglutinant.
- 1/5 en volume de solution aqueuse de bicarbonate d'ammonium à 0,1 mol.l$^{-1}$, à laquelle a été ajouté 10 mg/ml de fluorescéine de sodium, formant un marqueur fluorescent. Cela permet de suivre optiquement la dispersion par solubilisation et la diffusion du soluté 8 formé dans la chambre fluidique.
- 1/5 en volume de solution aqueuse de bicarbonate d'ammonium à 0,1 mol.l$^{-1}$, à laquelle a été ajouté 50 mg/ml de tréhalose. Le tréhalose agit en tant que sucre, assurant une protection du réactif agglutinant durant la déshydratation.
- 2/5 en volume de solution aqueuse de bicarbonate d'ammonium à 0,1 mol.l$^{-1}$.

La fonction du bicarbonate d'ammonium est une stabilisation du pH à une valeur proche de 8, permettant une bonne agglutination des globules rouges sous l'effet du réactif agglutinant anti-A.

La chambre fluidique 15 a fait l'objet d'un traitement préalable au plasma O$_2$ avec l'équipement de dépôt en phase vapeur MVD (marque déposée) 100 du fournisseur Applied Microstructures, pendant 600 secondes, avec un débit de 450 cm$^3$/min et une puissance de 200 W. Le composant microfluidique est réalisé en COP (acronyme de cyclo-olefin polymer), l'angle de contact de l'eau sur un tel polymère étant de 94°. Le plasma O$_2$ permet une diminution de l'angle de contact, ce dernier passant d'environ 90° avant traitement à 35° après traitement. On dispose alors d'une chambre fluidique 15 dont les paros internes sont hydrophiles. La solution de préparation, décrite ci-dessus, formant un solvant dans lequel est dissout un composé 8, est ensuite introduite dans la chambre fluidique. Cette dernière est ensuite séchée à température ambiante dans un dessiccateur branché sur une pompe à vide pendant 12 heures. Un composé 8 se forme au niveau des arêtes de la chambre, comme représenté sur la figure 1D. Il comporte notamment le tréhalose, la fluorescéine de sodium, et le réactif d'agglutination à l'état sec.

Dans une première série d'essais, on utilise un premier échantillon formé par des billes de polystyrène de diamètre 10 μm baignant dans un tampon salin PBS (acronyme de Phosphate Buffer Saline signifiant tampon phosphate salin), la fraction volumique étant de 0,1%. L'observation est réalisée avec un microscope, selon une configuration représentée sur la figure 2A, en utilisant un objectif 16 de grossissement x5. Les images ont été acquises à l'aide d'un capteur d'image couleur CMOS Mightex BCE-C050-U. La source de lumière 11 utilisée est une source de lumière blanche à halogène. La chambre fluidique 15 est disposée horizontalement entre la source de lumière et l'objectif, de telle sorte que le plan XZ représenté sur la figure 1C soit horizontal.

On a tout d'abord utilisé un composant fluidique dont la chambre 15 n'a pas subi de phase préalable de dépôt d'un composé selon une zone de dépôt délimitée. Les figures 6A, 6B, 6C, 6D et 6E sont des images respectivement acquises 20 s, 45 s, 70 s, 95 s et 120 secondes après l'introduction du premier échantillon dans la chambre fluidique 15. Sur ces images, on note une répartition aléatoire des particules dans l'échantillon, sans évolution marquée en fonction du temps.

Au cours d'un deuxième essai, on a ensuite utilisé une chambre fluidique 15 ayant subi une phase préalable de dépôt d'un composé sec selon la solution de préparation décrite ci-dessus. Comme on peut l'observer sur la figure 1D, la chambre fluidique 15 comporte un dépôt de composé sec 8 le long de l'extrémité inférieure 15i, cette dernière formant une arête. Les figures 7A, 7B, 7C, 7D et 7E sont des images respectivement acquises 20 s, 45 s, 70 s, 95 s et 120 secondes après l'introduction du premier échantillon dans la chambre fluidique, cette dernière étant orientée comme dans le premier essai (plan XZ horizontal). Sur ces images, on observe une zone grise s'étendant progressivement dans l'échantillon, à partir de l'extrémité inférieure 15i. Cela correspond à la diffusion du composé sec 8 dans la chambre fluidique 15. On observe également l'apparition d'une partie appauvrie 10d, à proximité de l'extrémité inférieure 15i, en particulier sur les figures 7C à 7E.

Au cours d'un troisième essai, on a utilisé des échantillons comportant du sang. Les figures 8A, 8B, 8C, 8D et 8E sont des images respectivement acquises 20 s, 45 s, 70 s, 95 s et 120 secondes après l'introduction d'un échantillon 10 formé de sang dilué au 1/5$^{ième}$, dans la chambre fluidique 15. La chambre fluidique 15 est orientée comme dans les essais précédents (plan XZ horizontal). Le sang provient d'un donneur de groupe B, si bien qu'il n'y a pas d'agglutination. On observe une structuration de l'échantillon dans la zone d'analyse ZA, sous l'effet de la diffusion du soluté : une partie appauvrie se forme, et s'étend à partir de l'extrémité inférieure 15i. La partie appauvrie 10d est essentiellement constituée de plasma sanguin, et présente une transmission de l'onde lumineuse incidente 12 élevée, d'où son aspect clair sur les figures. Une partie enrichie 10e se forme, en étant adjacente à la partie appauvrie 10d. On observe que la partie appauvrie 10d s'étend progressivement, ce qui entraîne un décalage progressif de la partie enrichie 10e.

Les figures 9A, 9B, 9C, 9D et 9E sont des images respectivement acquises 20 s, 45 s, 70 s, 95 s et 120 secondes après l'introduction d'un échantillon 10, formé de sang total dans la chambre fluidique 15. La chambre fluidique est orientée comme dans les essais précédents (plan XZ horizontal). Durant ce quatrième essai, le sang provient d'un donneur de groupe B, si bien qu'il n'y a pas d'agglutination. De même que dans les figures précédentes, on observe une structuration de l'échantillon, avec la formation d'une partie appauvrie 10d à partir de l'extrémité inférieure 15i, et la formation d'une partie enrichie 10e adjacente de la partie appauvrie 10d. Par rapport aux figures 8A à 8E, la partie enrichie apparaît plus foncée du fait de la concentration plus importante de globules rouges, le sang n'étant pas dilué.

Les figures 10A, 10B, 10C, 10D et 10E sont des images respectivement acquises 20 s, 45 s, 70 s, 95 s et 120 secondes après l'introduction d'un échantillon 10, formé de sang total d'un donneur de groupe A, dans la chambre fluidique 15. La chambre fluidique est orientée comme dans les essais précédents (plan XZ horizontal). Durant ce cinquième essai, et de même que dans les essais 2 à 4, on observe une structuration de l'échantillon, avec la formation d'une partie appauvrie 10d s'étendant à partir de l'extrémité inférieure 15i, et la formation d'une partie enrichie 10e adjacente de la partie appauvrie 10d. Par rapport aux figures 9A à 9E, l'aspect de la partie enrichie 10e est différent, du fait de l'agglomération des globules rouges sous l'effet du réactif d'agglutination anti-A.

Dans les images acquises au cours des essais 3 à 5, la formation d'une partie appauvrie 10d permet la sélection, sur chaque image acquise, d'une première région d'intérêt $ROI_1$ correspondant à la partie appauvrie 10d de l'échantillon. Une telle région d'intérêt permet de caractériser le plasma. De façon complémentaire ou alternative, il est possible de sélectionner une deuxième région d'intérêt $ROI_2$, correspondant à la partie enrichie 10e de l'échantillon. Une telle région d'intérêt permet de caractériser les particules , par exemple leur agglutination, ou le ralentissement, voire l'arrêt, de leur mouvement lors d'une coagulation.

Durant un sixième d'essai, on a mis en œuvre un dispositif d'analyse tel que celui représenté sur la figure 2A, le composant fluidique utilisé étant le celui représenté sur les figures 1A à 1D, maintenu verticalement, de telle sorte que l'axe longitudinal X soit parallèle à la gravité. Ainsi, le plan XZ est vertical. L'objectif 16 est un objectif Navitar 1-60135, monté par rapport au composant fluidique de façon à obtenir un grossissement voisin de 5. La source de lumière 11 est une diode électroluminescente blanche. Le capteur d'image 20 est un capteur CMOS Mightex tel que précédemment décrit. Les figures 11A, 11B, 11C, 11D et 11E sont des images respectivement acquises 20 secondes, 45 secondes, 70 secondes, 95 secondes et 120 secondes après l'introduction d'un échantillon, formé de sang total prélevé sur un donneur de groupe B, dans la chambre fluidique 15. De même que dans les figures précédentes, on observe une structuration de l'échantillon, avec la formation d'une partie appauvrie 10d adjacente de l'extrémité inférieure 15i, et d'une partie enrichie 10e adjacente de la partie appauvrie 10d.

Les figures 12A, 12B, 12C, 12D et 12E sont des images respectivement acquises 20 secondes, 45 secondes, 70 secondes, 95 secondes et 120 secondes après l'introduction d'un échantillon dans la chambre fluidique 15. Au cours de ce septième essai, l'échantillon est formé de sang total prélevé sur un donneur de groupe A, les chambre fluidique 15 étant disposée de façon similaire au sixième essai. De même que dans les essais 2 à 6, on observe une structuration de l'échantillon, avec la formation d'une partie appauvrie 10d adjacente de l'extrémité inférieure 15i, et une partie enrichie 10e adjacente de la partie appauvrie 10d. Les images de cette série ont un aspect différent des figures obtenues lors de l'essai précédent, du fait de l'agglutination des globules rouges sous l'effet du réactif anti-A.

Dans ces images, il est possible de sélectionner une première région d'intérêt $ROI_1$ dans une partie appauvrie 10d, de façon à caractériser le plasma. Il est également possible de sélectionner une deuxième région d'intérêt $ROI_2$, dans une partie enrichie 10e, de façon à caractériser une propriété des particules, par exemple une densité ou un niveau d'agglutination.

Au cours d'un huitième essai, on a utilisé le composant fluidique décrit en lien avec les sixième et septième essais pour déterminer une évolution d'un indicateur d'agglutination *ind* des particules, permettant d'identifier le groupe sanguin du sang introduit. La chambre fluidique 15 a été positionnée de la même façon que lors des sixième et septième essais, l'axe longitudinal X étant orienté selon la gravité, le plan XZ étant vertical. Différentes images ont été acquises, selon une fréquence d'acquisition de 7 images par seconde, et on a déterminé un indicateur de corrélation spatiale *ind* entre deux images successives. Un tel indicateur est décrit dans l'expression (4) du brevet européen EP2669678.

Si $I_n$ désigne une image acquise à un instant $n$ et $I_{n+m}$ désigne une image acquise à un instant $n + m$, on peut former deux images $A_n$ et $A_{n+m}$ telles que :

$A_n = I_n - (I_n * k_1)$ et $A_{n+m} = I_{n+m} - (I_{n+m} * k_1)$, le symbole * désignant l'opérateur produit de convolution. $k_1$ est un noyau de convolution carré de 11 pixels de côtés, formé de valeurs égales à $1/11^2$. Les images $(I_n * k_1)$ et $(I_{n+m} * k_1)$ correspondent respectivement à des moyennes locales des images $I_n$ et $I_{n+m}$. On peut former une image de corrélation $Icorr_{n,n+m}$, dont chaque pixel $Icorr_{n,n+m}(x,y)$ exprime une corrélation entre les images $I_n$ et $I_{n+m}$ au niveau dudit pixel. Cette image de corrélation peut être obtenue selon l'expression suivante :

$$Icorr_{n,n+m} = \frac{(A_n \times A_{n+m}) * k_1}{\sqrt{((A_n)^2 * k_1)}\sqrt{((A_{n+m})^2 * k_1)}}$$

où x représente le produit matriciel de Hadamard. L'indicateur d'agglutination *Ind* est ensuite calculé en fonction de l'intensité des pixels de l'image de corrélation *Icorr$_{n,n+m}$*, par exemple en effectuant une moyenne. Il représente une corrélation entre les images *I$_n$* et *I$_{n+m}$*. Dans cet exemple, m = 21. L'indicateur d'agglutination représente une corrélation entre deux images *I$_n$* et *I$_{n+m}$* temporellement décalées de 3 secondes.

La figure 13A représente un exemple d'image obtenue, cette image correspondant la zone d'analyse ZA représentée sur la figure 1C. Sur cette image, on a représenté trois régions d'intérêt ROI$_a$, ROI$_b$ et ROI$_c$. La région d'intérêt ROI$_a$ correspond à une partie enrichie 10e de l'échantillon tandis que la région d'intérêt ROI$_b$ correspond à une zone périphérique de la partie enrichie et la région d'intérêt ROI$_c$ correspond à une partie appauvrie 10d de l'échantillon.

La figure 13B représente des indicateurs d'agglutination *ind* déterminés, en fonction du temps, en considérant la région d'intérêt ROI$_a$, pour 4 échantillons : 2 échantillons de sang humain de groupe A (croix) et 2 échantillons de sang humain de groupe B (cercles). Sur les échantillons positifs (groupe sanguin A), l'indicateur d'agglutination *ind* augmente régulièrement et atteint un plateau, correspondant à un indicateur de corrélation élevé. Cette corrélation élevée est une signature d'une agglutination des globules rouges, ces derniers s'immobilisant peu à peu dans la chambre fluidique. Au contraire, avec les échantillons négatifs (groupe sanguin B), la corrélation tend à fluctuer et reste faible, du fait des mouvements persistants des globules rouges dans la chambre fluidique en l'absence de coagulation. L'indicateur *Ind* varie mais reste généralement faible.

La figure 13C représente des indicateurs d'agglutination *Ind* déterminés, en fonction du temps, en considérant la région d'intérêt ROI$_b$, pour les 4 échantillons décrits en lien avec la figure 13B.

On observe que les courbes obtenues pour les échantillons négatifs (cercles) comportent des fluctuations nettement plus importantes que dans le cas précédent. La figure 13D représente des indicateurs d'agglutination *Ind* déterminés, en fonction du temps, en considérant la région d'intérêt ROI$_c$, pour les 4 échantillons décrits en lien avec la figure 13B. On observe que les courbes obtenues pour les échantillons négatifs (cercles) comportent des fluctuations, tandis que les courbes correspondant aux échantillons positifs (croix) ne sont pas représentatives de l'immobilisation progressive des particules sous l'effet de la coagulation.

Ainsi, la sélection d'une région d'intérêt ROI adaptée au phénomène observé dans la chambre fluidique a un impact sur la caractérisation de l'échantillon. Lorsque la caractérisation est liée à une agglomération des particules ou une coagulation de l'échantillon, la sélection d'une région d'intérêt dans une partie enrichie 10e, ou dans une partie rapide 10f, est préférable pour obtenir une caractérisation plus précise.

Un neuvième essai a été réalisé en mettant en œuvre un composant fluidique similaire à celui représenté sur la figure 4A, la chambre fluidique 15 étant maintenue horizontalement, face à l'objectif de microscope précédemment décrit (plan XY horizontal). La chambre fluidique a préalablement subi un traitement au plasma oxygène, de façon à obtenir une chambre fluidique dont les parois internes sont hydrophiles. La solution de préparation telle que précédemment décrite a été introduite dans la chambre fluidique. Cette dernière a ensuite a fait l'objet d'un séchage. Au cours du séchage, des dépôts de composés secs 8 se sont formés au niveau des arêtes parois latérales 15$_1$ et 15$_2$, formant des zones de dépôt ZD, comme schématisé sur la figure 4B.

Les figures 14A, 14B, 14C, 14D, 14E et 14F représentent des images de la chambre fluidique 15 acquises respectivement au moment de l'introduction d'un tampon PBS comportant des billes de polystyrène, puis 15 secondes, 30 secondes, 45 secondes, 60 secondes et 75 secondes après l'introduction de l'échantillon. Les particules de polystyrène ont un diamètre de 10 $\mu$m et leur concentration en volume est de 5%. Au niveau de chaque paroi latérale 15$_1$ et 15$_2$, on observe une partie appauvrie 10d s'élargissant progressivement. En bordure de chaque partie appauvrie, on observe la formation progressive d'une partie enrichie 10e. La segmentation de la chambre fluidique en une partie appauvrie 10d et une partie enrichie 10e est plus marquée au voisinage de la paroi latérale 15$_1$ probablement du fait d'une quantité plus importante de composé sec 8 formé sur la zone de dépôt ZD correspondant à cette paroi.

La figure 14G représente un dixième essai, similaire au neuvième essai, la concentration des particules étant de 0.1% en volume. On a acquis cette image 60 secondes après l'introduction de l'échantillon dans la chambre fluidique. On peut observer la formation d'une partie appauvrie 10d au niveau de chaque paroi latérale 15$_1$ et 15$_2$, ainsi qu'une partie enrichie 10e s'étendant entre les zones appauvries.

La figure 15A représente un onzième essai, dans lequel on a introduit un tampon PBS comportant des billes de polystyrène de diamètre 10 $\mu$m, dans une chambre fluidique telle que représentée sur la figure 4A. La chambre fluidique a fait l'objet d'une préparation similaire à celle décrite en lien avec le neuvième essai, de façon à obtenir une zone de dépôt ZD s'étendant le long des parois latérales 15$_1$ et 15$_2$. La concentration en volume est égale à 0.1%. La chambre fluidique 15 a été maintenue verticalement, la gravité étant orientée selon l'axe longitudinal X, ce dernier correspondant à la direction de progression de l'échantillon dans la chambre fluidique. Ainsi, le plan XY est vertical. La figure 15A est obtenue par addition de 21 images acquises par le capteur d'image, à une fréquence d'acquisition de 7 images par secondes. Aussi, chaque particule apparaît non pas sous la forme de points, mais de traces, dont la longueur est représentative de la distance parcourue par la particule en 3 secondes. Ces images ont été obtenues 5 secondes après l'introduction de l'échantillon dans la chambre fluidique. Ainsi, la longueur de chaque trace dépend de la vitesse de la particule à correspondant à ladite trace.

On observe la formation de gradients de vitesses dans l'échantillon. Au voisinage de chaque zone de dépôt ZD, la vitesse est plus élevée, en étant orientée dans la direction de la gravité. Les inventeurs attribuent cet effet à une augmentation locale de la densité de l'échantillon, du fait de la concentration élevée en composé dispersé. Un courant se forme alors dans la chambre fluidique, entraînant l'échantillon selon l'axe de la gravité, au voisinage de chaque zone de dépôt. Il se forme alors des zones, dites zones rapides 10f, dans laquelle les particules ont une vitesse plus élevée que leur vitesse moyenne dans la chambre fluidique. En bordure de chaque partie rapide une partie lente 10s apparaît, dans laquelle les particules ont une vitesse plus lente que la vitesse moyenne des particules dans la chambre fluidique 15. Dans la partie médiane de la chambre fluidique, c'est-à-dire à égale distance entre chaque zone de dépôt, une partie rapide se forme, dans laquelle les particules se déplacent selon une direction opposée à l'axe longitudinal X. On comprend alors qu'une circulation s'établit dans la chambre fluidique, des particules se déplaçant selon l'axe longitudinal X au voisinage de chaque zone de dépôt, puis dans le sens inverse à l'axe longitudinal X, dans une partie de la chambre fluidique suffisamment éloignée de chaque zone de dépôt ZD. Sur la figure 15, le sens de déplacement des particules a été matérialisé par des flèches. La figure 15B représente un douzième essai, dans des conditions similaires au onzième essai représenté sur la figure 15A, les différences étant l'orientation de la chambre fluidique 15, le plan XY étant horizontal, ainsi que la concentration en billes de polystyrène, cette dernière étant de 5% en volume. On observe à la fois l'apparition de parties enrichies 10e et de parties appauvries 10d, ainsi que des parties rapides 10f et des parties lentes 10s.

La segmentation de la chambre fluidique 15 selon différentes vitesses de particules peut être mise à profit pour sélectionner, dans l'image acquise par le capteur d'image 20, une partie rapide 10f, en particulier pour quantifier une agglutination de particules ou une coagulation. En effet, le ralentissement des particules est d'autant mieux caractérisé dans les parties de l'échantillon dans lesquelles les particules ont une vitesse initiale élevée. Par vitesse initiale, on entend une vitesse après l'introduction de l'échantillon 10 dans la chambre fluidique 15, lorsque la réaction d'agglutination ou de coagulation n'a pas encore eu lieu.

Par ailleurs, on peut également caractériser une partie de l'échantillon 10c dite de cisaillement, s'étendant à l'interface d'une partie rapide 10f et d'une partie lente 10s, ou comportant deux parties rapides dans lesquelles le déplacement des particules se fait en sens opposé. Dans de telles parties, l'échantillon est soumis à un cisaillement, propice à l'observation de certains effets, par exemple la lyse de particules.

Quel que soit le mode de réalisation, les inventeurs ont constaté que pour obtenir une partie enrichie 10e et une partie appauvrie 10d se distinguant nettement, il était préférable que le gradient de concentration du composé ne soit pas parallèle à la gravité, et notamment ne soit pas dirigé selon le sens contraire à la gravité. Lorsque le gradient $\overline{grad}$ de concentration du composé est parallèle à la gravité, et orienté le sens contraire à la gravité, la séparation en une partie appauvrie 10d et une partie enrichie 10e peut être perceptible, mais est généralement moins marquée que dans les configurations dans lesquelles le gradient de composé est perpendiculaire à la gravité. Il en est de même pour la séparation entre une partie rapide 10f et une partie lente 10s. Leur observation est facilitée lorsque le gradient de concentration n'est ni parallèle à la gravité, ni orienté selon le sens contraire de la gravité. La séparation entre une partie rapide 10f et une partie lente 10s est optimale lorsque la gravité est orthogonale au gradient $\overline{grad}$ de concentration du composé 8.

Ainsi, l'invention permet de sélectionner une région d'intérêt ROI en fonction d'un critère de sélection, relatif à une concentration de particules (partie enrichie ou partie appauvrie) et/ou relatif à une vitesse des particules (partie lente ou partie rapide) dans la chambre fluidique 15. Chaque région d'intérêt ROI peut être déterminée sur la base d'une analyse d'image acquise par le capteur d'image, en considérant le critère pris en compte, l'analyse d'image permettant de déterminer les parties enrichies, appauvries, et/ou les parties lentes ou rapides. Chaque région d'intérêt ROI peut également être définie, sur la base d'essais de calibration. Un essai de calibration consiste à acquérir une ou plusieurs images d'un échantillon de calibration, considéré comme représentatif de l'échantillon à caractériser, et de définir la position d'une région d'intérêt en fonction du critère de sélection retenu. La région d'intérêt ainsi définie est ensuite appliquée aux images acquises des échantillons à caractériser.

L'invention pourra être mise en œuvre dans la caractérisation d'échantillons biologiques, comportant notamment des liquides ou des particules corporelles, à des fins d'aide au diagnostic. Elle pourra aussi être utilisée dans le domaine de l'environnement, de l'agroalimentaire ou dans le contrôle de procédés industriels.

## Revendications

1. Procédé de caractérisation d'un échantillon (10), l'échantillon comportant des particules baignant dans un milieu liquide, le procédé comportant les étapes suivantes :

   a) introduction de l'échantillon dans une chambre fluidique (15), la chambre fluidique comportant un composé (8) à l'intérieur de la chambre et s'étendant dans cette dernière en formant une zone de dépôt délimitée (ZD) ;
   b) dispersion, par l'échantillon, du composé (8), le composé ainsi dispersé diffusant dans la chambre fluidique (15) en établissant un gradient de concentration ($\overrightarrow{grad}$) s'étendant à partir de la zone de dépôt (ZD);

c) sous l'effet du gradient de concentration ($\overrightarrow{grad}$), mise en mouvement des particules dans la chambre fluidique durant un intervalle temporel de diffusion ($\Delta t$), de façon à former, dans la chambre fluidique :

- une partie enrichie en particules (10e) et une partie appauvrie en particules (10d) ;
- et/ou une partie dite rapide (10f) et une partie dite lente (10s), la vitesse des particules étant plus élevée dans la partie rapide que dans la partie lente ;

d) prise en compte d'un critère de sélection relatif à une concentration des particules ou à une vitesse des particules ;

e) illumination de la chambre fluidique (15) à l'aide d'une source de lumière (11) ;

f) acquisition d'une image (*I*) de la chambre fluidique (15), par un capteur d'image (20), durant l'intervalle temporel de diffusion ($\Delta t$), et sélection, dans l'image acquise (*I*), d'une région d'intérêt (ROI) dans laquelle les particules satisfont au critère de sélection;

g) caractérisation de l'échantillon (10) à partir de la région d'intérêt sélectionnée ; le procédé étant tel que :

- lors de l'étape d), le critère de sélection est une faible concentration de particules, auquel cas lors de l'étape f), la région d'intérêt est la partie (10d) appauvrie en particules de la chambre fluidique ;
- ou lors de l'étape d), le critère de sélection est une forte concentration de particules, auquel cas lors de l'étape f), la région d'intérêt est la partie (10e) enrichie en particules de la chambre fluidique;
- ou lors de l'étape d), le critère de sélection est une vitesse élevée de particules, auquel cas lors de l'étape f), la région d'intérêt est la partie rapide (10f) de la chambre fluidique ;
- ou lors de l'étape d), le critère de sélection est une faible vitesse de particules, auquel cas lors de l'étape f), la région d'intérêt est à la partie lente (10s) de la chambre fluidique ;
- ou lors de l'étape d), le critère de sélection est un cisaillement de l'échantillon, auquel cas, lors de l'étape f), la région d'intérêt est une partie dite de cisaillement (10c) s'étendant entre la partie lente (10s) et la partie rapide (10f) de la chambre fluidique, ou entre deux parties rapides dans lesquelles les particules se déplacent respectivement selon des vitesses de sens opposé.

2. Procédé selon la revendication 1, dans lequel la chambre fluidique (15) comporte une paroi interne hydrophile (15p), délimitée par une arête (15i), de telle sorte que la zone de dépôt (ZD) s'étend parallèlement à l'arête, et de telle sorte que lors de l'étape c), la partie appauvrie (10d) est adjacente de l'arête.

3. Procédé selon l'une quelconque des revendications 1 ou 2, dans lequel la chambre fluidique comporte une face interne sur laquelle est ménagé un motif hydrophile ou hydrophobe, de telle sorte que la zone de dépôt (ZD) corresponde au motif.

4. Procédé selon l'une quelconque des revendications précédentes, dans lequel un sillon est ménagé sur une face interne de la chambre fluidique (15), de telle sorte que la zone de dépôt (ZD) corresponde au sillon.

5. Procédé selon l'une quelconque des revendications précédentes, dans lequel :

- la partie enrichie (10e) est adjacente de la partie appauvrie (10d), la partie appauvrie s'étendant entre la partie enrichie et la zone de dépôt (ZD) ;

ou

- la partie lente (10s) est adjacente de la partie rapide (10f), la partie rapide (10f) s'étendant entre la partie lente et la zone de dépôt (ZD).

6. Procédé selon l'une quelconque des revendications précédentes, dans lequel l'intervalle temporel de diffusion ($\Delta t$) est supérieur à 10 secondes, voire à 30 secondes et/ou inférieur à 20 minutes.

7. Procédé selon l'une quelconque des revendications précédentes, dans lequel la chambre fluidique (15) comporte un réactif d'analyse apte à favoriser une agglutination des particules, ou un réactif apte à favoriser une coagulation de l'échantillon, ou un réactif apte à colorer l'échantillon ou à un réactif apte à entraîner une fluorescence de l'échantillon.

8. Procédé selon la revendication 7, dans lequel le réactif d'analyse est compris dans le composé (8).

9. Procédé selon l'une quelconque des revendications 7 ou 8, dans lequel le réactif d'analyse est apte :

- à favoriser une agglutination des particules ou une coagulation de l'échantillon, la région d'intérêt (ROI) sélectionnée lors de l'étape f) étant une partie enrichie (10e) de la chambre fluidique ou une partie rapide (10f) de la chambre fluidique;
- et/ou à favoriser une coloration du milieu liquide de l'échantillon, la région d'intérêt sélectionnée lors de l'étape f) étant une partie appauvrie (10d) de la chambre fluidique;
- et/ou à favoriser une coloration ou une fluorescence des particules de l'échantillon, la région d'intérêt sélectionnée lors de l'étape f) étant une partie enrichie de la chambre fluidique.

10. Procédé selon l'une quelconque des revendications précédentes, comportant, préalablement à l'étape a) :

- un remplissage de la chambre fluidique par un liquide (9), dit liquide porteur (8), dans lequel le composé est dispersé ;
- un séchage du liquide porteur de façon à former un dépôt sec de composé (8) suite à l'évaporation du liquide porteur.

11. Procédé selon l'une quelconque des revendications précédentes, dans lequel la position de la région d'intérêt (ROI) est déterminée lors d'une phase de calibration, à l'aide d'échantillons de calibration, en fonction d'un critère de sélection correspondant au critère de sélection pris en compte dans l'étape d).

**Patentansprüche**

1. Verfahren zur Charakterisierung einer Probe (10), wobei die Probe Partikel umfasst, die in einem flüssigen Medium schwimmen, wobei das Verfahren die folgenden Schritte umfasst:

a) Einführen der Probe in eine Fluidkammer (15), wobei die Fluidkammer eine Verbindung (8) im Inneren der Kammer umfasst und sich in dieser Letzteren erstreckt, indem sie eine begrenzte Aufgabezone (ZD) bildet;
b) Dispergieren, durch die Probe, der Verbindung (8), wobei die so dispergierte Verbindung in die Fluidkammer (15) diffundiert, wobei sie einen Konzentrationsgradienten ($\overrightarrow{grad}$) aufbaut, der sich von der Aufgabezone (ZD) aus erstreckt;
c) unter der Wirkung des Konzentrationsgradienten ($\overrightarrow{grad}$), Inbewegungversetzen der Partikel in der Fluidkammer während eines Diffusionszeitintervalls ($\Delta t$) derart, dass in der Fluidkammer gebildet werden:

- ein an Partikeln angereicherter Teil (10e) und ein an Partikeln verarmter Teil (10d);
- und/oder ein sogenannter schneller Teil (10f) und ein sogenannter langsamer Teil (10s), wobei die Geschwindigkeit der Partikel im schnellen Teil höher als im langsamen Teil ist;

d) Berücksichtigen eines Auswahlkriteriums bezüglich einer Konzentration der Partikel oder einer Geschwindigkeit der Partikel;
e) Beleuchten der Fluidkammer (15) mithilfe einer Lichtquelle (11);
f) Erfassen eines Bildes ($I$) der Fluidkammer (15) durch einen Bildsensor (20) während des Diffusionszeitintervalls ($\Delta t$) und Auswählen eines relevanten Bereichs (ROI) in dem erfassten Bild ($I$), in dem die Partikel das Auswahlkriterium erfüllen;
g) Charakterisieren der Probe (10) anhand des ausgewählten relevanten Bereichs; wobei das Verfahren dergestalt ist, dass:

- beim Schritt d) das Auswahlkriterium eine schwache Partikelkonzentration ist, wobei in dem Fall beim Schritt f) der relevante Bereich der an Partikeln verarmte Teil (10d) der Fluidkammer ist;
- oder beim Schritt d) das Auswahlkriterium eine starke Partikelkonzentration ist, wobei in dem Fall beim Schritt f) der relevante Bereich der an Partikeln angereicherte Teil (10e) der Fluidkammer ist;
- oder beim Schritt d) das Auswahlkriterium eine hohe Partikelgeschwindigkeit ist, wobei in dem Fall beim Schritt f) der relevante Bereich der schnelle Teil (10f) der Fluidkammer ist;
- oder beim Schritt d) das Auswahlkriterium eine geringe Partikelgeschwindigkeit ist, wobei in dem Fall beim Schritt f) der relevante Bereich der langsame Teil (10s) der Fluidkammer ist;

- oder beim Schritt d) das Auswahlkriterium eine Scherung der Probe ist, wobei in dem Fall beim Schritt f) der relevante Bereich ein sogenannter Scherungsteil (10c) ist, der sich zwischen dem langsamen Teil (10s) und dem schnellen Teil (10f) der Fluidkammer erstreckt oder zwischen zwei schnellen Teilen, in denen sich die Partikel jeweils mit entgegengesetzt gerichteten Geschwindigkeiten bewegen.

2. Verfahren nach Anspruch 1, bei dem die Fluidkammer (15) eine hydrophile Innenwand (15p) umfasst, die durch eine Kante (15i) begrenzt wird, so dass sich die Aufgabezone (ZD) parallel zu der Kante erstreckt und so, dass beim Schritt c) der verarmte Teil (10d) an die Kante angrenzt.

3. Verfahren nach einem der Ansprüche 1 oder 2, bei dem die Fluidkammer eine Innenseite umfasst, auf der ein hydrophiles oder hydrophobes Muster ausgebildet ist, so dass die Aufgabezone (ZD) dem Muster entspricht.

4. Verfahren nach einem der vorhergehenden Ansprüche, bei dem eine Furche auf einer Innenseite der Fluidkammer (15) ausgebildet ist, so dass die Aufgabezone (ZD) der Furche entspricht.

5. Verfahren nach einem der vorhergehenden Ansprüche, bei dem:

   - der angereicherte Teil (10e) an den verarmten Teil (10d) angrenzt, wobei sich der verarmte Teil zwischen dem angereichten Teil und der Aufgabezone (ZD) erstreckt;

   oder

   - der langsame Teil (10s) an den schnellen Teil (10f) angrenzt, wobei sich der schnelle Teil (10f) zwischen dem langsamen Teil und der Aufgabezone (ZD) erstreckt.

6. Verfahren nach einem der vorhergehenden Ansprüche, bei dem das Diffusionszeitintervall ($\Delta$t) mehr als 10 Sekunden oder sogar als 30 Sekunden und/oder weniger als 20 Minuten beträgt.

7. Verfahren nach einem der vorhergehenden Ansprüche, bei dem die Fluidkammer (15) ein Analysereagens umfasst, das geeignet ist, eine Agglutination der Partikel zu begünstigen, oder ein Reagens, das geeignet ist, eine Koagulation der Probe zu begünstigen, oder ein Reagens, das geeignet ist, die Probe zu färben, oder ein Reagens, das geeignet ist, eine Fluoreszenz der Probe herbeizuführen.

8. Verfahren nach Anspruch 7, bei dem das Analysereagens in der Verbindung (8) enthalten ist.

9. Verfahren nach einem der Ansprüche 7 oder 8, bei dem das Analysereagens geeignet ist:

   - eine Agglutination der Partikel oder eine Koagulation der Probe zu begünstigen, wobei der beim Schritt f) gewählte relevante Bereich (ROI) ein angereicherter Teil (10e) der Fluidkammer oder ein schneller Teil (10f) der Fluidkammer ist;
   - und/oder eine Färbung des flüssigen Mediums der Probe zu begünstigen, wobei der beim Schritt f) gewählte relevante Bereich ein verarmter Teil (10d) der Fluidkammer ist;
   - und/oder eine Färbung oder eine Fluoreszenz der Partikel der Probe zu begünstigen, wobei der beim Schritt f) gewählte relevante Bereich ein angereicherter Teil der Fluidkammer ist.

10. Verfahren nach einem der vorhergehenden Ansprüche, das vor dem Schritt a) umfasst:

    - ein Füllen der Fluidkammer mit einer Flüssigkeit (9), Trägerflüssigkeit (8) genannt, in der die Verbindung dispergiert ist;
    - ein Trocknen der Trägerflüssigkeit, so dass nach dem Verdampfen der Trägerflüssigkeit ein trockenes Verbindungsdepot (8) gebildet wird.

11. Verfahren nach einem der vorhergehenden Ansprüche, bei dem die Position des relevanten Bereichs (ROI) bei einer Kalibrierungsphase mithilfe von Kalibrierungsproben in Abhängigkeit von einem Auswahlkriterium bestimmt wird, das dem in Schritt d) berücksichtigten Auswahlkriterium entspricht.

**Claims**

1.  Method for characterizing a sample (10), the sample comprising particles bathing in liquid medium, the process comprising the following stages:

    a) introduction of the sample into a fluid chamber (15), the fluid chamber comprising a compound (8) inside the chamber and spreading out in the latter, forming a delimited zone of deposition (ZD);
    b) dispersion, by the sample, of the compound (8), the compound thus dispersed diffusing in the fluid chamber (15), establishing a concentration gradient ($\overrightarrow{grad}$) extending from the zone of deposition (ZD);
    c) under the effect of the concentration gradient ($\overrightarrow{grad}$), placing the particles in movement in the fluid chamber for a diffusion time interval ($\Delta t$), so as to form, in the fluid chamber:

    - a part enriched in particles (10e) and a part depleted in particles (10d);
    - and/or a "rapid" part (10f) and a "slow" part (10s), the velocity of the particles being higher in the rapid part than in the slow part;

    d) taking into account a selection criterion relating to a concentration of the particles or to a velocity of the particles;
    e) illumination of the fluid chamber (15) using a light source (11);
    f) acquisition of an image (/) of the fluid chamber (15) by an image sensor (20) during the diffusion time interval ($\Delta t$) and selection, in the acquired image (/), of a region of interest (ROI) in which the particles satisfy the selection criterion;
    g) characterization of the sample (10) from the region of interest selected; the process being such that:

    - during stage d), the selection criterion is a low concentration of particles, in which case, during stage f), the region of interest is the part (10d) of the fluid chamber depleted in particles;
    - or, during stage d), the selection criterion is a high concentration of particles, in which case, during stage f), the region of interest is the part (10e) of the fluid chamber enriched in particles;
    - or, during stage d), the selection criterion is a high velocity of particles, in which case, during stage f), the region of interest is the rapid part (10f) of the fluid chamber;
    - or, during stage d), the selection criterion is a low velocity of particles, in which case, during stage f), the region of interest is the slow part (10s) of the fluid chamber;
    - or, during stage d), the selection criterion is a shearing of the sample, in which case, during stage f), the region of interest is a "shearing" part (10c) extending between the slow part (10s) and the rapid part (10f) of the fluid chamber, or between two rapid parts in which the particles respectively move according to velocities of opposite direction.

2.  Method according to Claim 1, in which the fluid chamber (15) comprises a hydrophilic internal wall (15p), delimited by a ridge (15i), so that the zone of deposition (ZD) extends parallel to the ridge and so that, during stage c), the depleted part (10d) is adjacent to the ridge.

3.  Method according to either one of Claims 1 and 2, in which the fluid chamber comprises an internal face on which is arranged a hydrophilic or hydrophobic pattern, so that the zone of deposition (ZD) corresponds to the pattern.

4.  Method according to any one of the preceding claims, in which a groove is arranged on an internal face of the fluid chamber (15) so that the zone of deposition (ZD) corresponds to the groove.

5.  Method according to any one of the preceding claims, in which:

    - the enriched part (10e) is adjacent of the depleted part (10d), the depleted part extending between the enriched part and the zone of deposition (ZD);

    or

    - the slow part (10s) is adjacent of the rapid part (10f), the rapid part (10f) extending between the slow part and the zone of deposition (ZD).

6.  Method according to any one of the preceding claims, in which the diffusion time interval ($\Delta t$) is greater than 10 seconds, indeed even than 30 seconds, and/or less than 20 minutes.

7.  Method according to any one of the preceding claims, in which the fluid chamber (15) comprises an analytical reagent capable of promoting an agglutination of the particles, or a reagent capable of promoting a coagulation of the sample, or reagent capable of colouring the sample, or a reagent capable of bringing about a fluorescence of the sample.

8.  Method according to Claim 7, in which the analytical reagent is comprised in the compound (8).

9.  Method according to either one Claims 7 and 8, in which the analytical reagent is capable:

    - of promoting an agglutination of the particles or a coagulation of the sample, the region of interest (ROI) selected during stage f) being an enriched part (10e) of the fluid chamber or a rapid part (10f) of the fluid chamber;
    - and/or of promoting a colouring of the liquid medium of the sample, the region of interest selected during stage f) being a depleted part (10d) of the fluid chamber;
    - and/or of promoting a colouring or a fluorescence of the particles of the sample, the region of interest selected during stage f) being an enriched part of the fluid chamber.

10. Method according to any one of the preceding claims, comprising, prior to stage a):

    - filling of the fluid chamber by a liquid (9), referred to as carrier liquid (8), in which the compound is dispersed;
    - drying of the carrier liquid so as to form a dry deposit of compound (8) following evaporation of the carrier liquid.

11. Method according to any one of the preceding claims, in which the position of the region of interest (ROI) is determined during a calibration phase, using calibration samples, according to a selection criterion corresponding to the selection criterion taken into account in stage d).

0.75

0.5

15s

15c

0.15

15p

15p

1.7

15i

0.3

**Fig. 1B**

15s

15

15c

15in

15i

**Fig. 1A**

20

6

15c

15s

ZA

15in

10

6.1

15i

**Fig. 1C**

15c

15s

ZD

15in

8

ZD

15i

**Fig. 1D**

**Fig. 2A**

**Fig. 2B**

**Fig. 2C**

**Fig. 3A**

**Fig. 3B**

**Fig. 3C**

**Fig. 3D**

**Fig. 3E**

ZD

15

**Fig. 3F**

$15_p$  $15_1$  15  15in

$15_2$  ZA

z
x
y

**Fig. 4A**

ZD  ZA  ZD

8  8

$15_1$  $15_2$

**Fig. 4B**

ZD  ZA  ZD

$15_1$

grad  grad

$15_2$

10d 10e  10e 10d

**Fig. 4C**

80 → 90 → 100

110

I

120

ROI

130

**Fig. 5**

ind

**Fig. 6A**  ZD  x  z  15i

**Fig. 6B**

**Fig. 6C**

**Fig. 6D**

**Fig. 6E**

**Fig. 7A**  ZD  15i

**Fig. 7B**

**Fig. 7C**  10e  10d

**Fig. 7D**  10e  10d

**Fig. 7E**  10e  10d  15i

**Fig. 8A** 15i    **Fig. 8B**    **Fig. 8C**

ZD

z
x

ROI₂

10e

**Fig. 8D**    ROI₁   **Fig. 8E**   10d

10e

10d

**Fig. 9A** 15i ZD    **Fig. 9B**    **Fig. 9C**

z
x

ROI₂

10e

**Fig. 9D**    ROI₁   **Fig. 9E**   10d

10e

10d

**Fig. 10A** 15i ZD    **Fig. 10B**    **Fig. 10C**

z
x

10e

10d

ROI₂

10e

**Fig. 10D**    ROI₁   **Fig. 10E**   10d

**Fig. 11A**

**Fig. 11B**

**Fig. 11C**

} 10e

} 10d

15i

ZD

**Fig. 11D**

} 10e

} 10d

**Fig. 11E**

ROI₂

} 10e

} 10d

ROI₁

**Fig. 12A**

15i

ZD

**Fig. 12B**

**Fig. 12C**

} 10e

} 10d

**Fig. 12D**

} 10e

} 10d

**Fig. 12E**

ROI₂

} 10e

} 10d

ROI₁

ROIb

**Fig. 13A**

**Fig. 13B**

**Fig. 13C**

**Fig. 13D**

Fig. 14A   Fig. 14B   Fig. 14C

Fig. 14D   Fig. 14E   Fig. 14F

Fig. 14G

Fig. 15B

Fig. 15A

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2233923 A
- US 20150301018 A
- EP 2669678 A
- US 2015160244 A
- EP 3076156 A
- EP 2964090 A
- WO 2016139409 A

**Littérature non-brevet citée dans la description**

- **HUET M.** Real time observation and automated measurement of red blood cells agglutination inside a passive microfluidic biochip containing embedded reagents. *Biosensors and Bioelectronics,* Septembre 2016
- **ANDERSON JOHN.** Diffusiophoresis : Migration of Colloidal Particles in Gradients of Solute Concentration. *Separation and Purification Reviews,* vol. 13 (1), 67-103
- **ABÉCASSIS B.** Osmotic manipulation of particles for microfluidic applications. *New J. Phys,* vol. 11, 075022